# EUROPEAN PATENT APPLICATION

(11) **EP 2 886 550 A1**
(43) Date of publication of application: **24.06.2015**
(21) Application number: 13306843.7
(22) Date of filing: 23.12.2013
(51) Int. Cl.: C07K 1/02, C07K 1/04

(54) **Dynamic molecules and dynamic combinatorial library**

(71) Applicant: Centre National de la Recherche Scientifique (CNRS), 75016 Paris (FR); Université de Strasbourg, 67000 Strasbourg (FR)
(72) Inventor: Ruff, Yves, 67000 Strasbourg (FR); Giuseppone, Nicolas, 67980 Hangenbieten (FR); Garavini, Valentina, 67000 Strasbourg (FR)
(74) Representative: Blot, Philippe Robert Emile

(57) **Abstract**

The invention relates to a mixture of molecules comprising at least molecules (p1 p2) as defined by formula (I) and at least molecules (p3) as defined by formula (II) 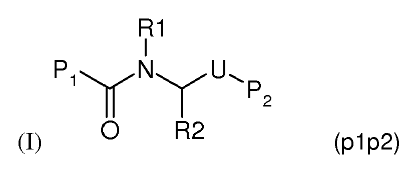 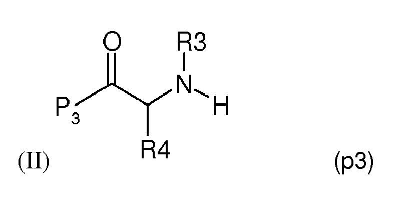 wherein R1, R2, R3, R4, P1, P2, and P3 are each independently atomic groups,
wherein at least one of R1 and R2 is -CH2(CH2)ₓ-SH wherein x is 0, or 1 to 6,
wherein at least one of R3 and R4 is -CH2(CH2)ₓ-SH wherein x is 0, or 1 to 6,
wherein -U- is aliphatic optionally substituted, for example -C(RaRb)-, wherein Ra and Rb are atomic groups, or aromatic optionally substituted, or -C(O)-, -O-, -S-, or a phosphorus containing function.

The invention also relates to such molecules, their applications, notably for preparing a combinatorial library or dynamic moelcules.

## Description

The present invention concerns molecules and mixture of molecules enabling the preparation of a combinatorial library. In particular, the invention relates to the preparation of dynamic molecules and preparation thereof for the purpose of synthetizing a dynamic combinatorial library.

### Technological background

With a hydrolysis half-time of 7 years in neutral water, the covalent peptide bond is practically inert under physiological conditions. Nevertheless, in living systems, the fast and selective modulation of protein expression and degradation in response to environmental changes requires to continuously form (e.g. ribosome) and break (e.g. proteases) peptide bonds. Typically, the average half-life time of a protein in cells ranges from 1 hour to 1 day. Most proteins and peptides are thus highly dynamic in their constitution, this property being instrumental in their biological functions.

Recently, synthetic chemists have tried to develop dynamic combinatorial libraries (DCLs) (E. Moulin, G. Cormos, N. Giuseppone, Chem. Soc. Rev. 2012, 41, 1031-1049 ; N. Giuseppone, Acc. Chem. Res. 2012, 45, 2178-2188 ; F. B. L. Cougon, J. K. M. Sanders Acc. Chem. Res. 2012, 45, 2211-2221; Constitutional Dynamic Chemistry, Top. Curr. Chem. 2012, 322, M. Barboiu Editor, Springer; M. Barboiu, Chem. Commun. 2010, 46, 7466-7476 ; J. Li, P. Nowak, S. Otto, J. Am. Chem. Soc. 2013, 135, 9222-9239) based on the exchange of peptide fragments. In a couple of examples, they succeeded in amplifying ligands of protein receptors (P. G. Swann, R. A. Casanova, A. Desai, M. M. Frauenhoff, M. Urbancic, U. Slomczynska, A. J. Hopfinger, G. C. Le Breton, D. L. Venton, Biopolymers 1996, 40, 617-625) and in sorting self-assembled dipeptide-based gelators under thermodynamic control (J. W. Sadownik, R. V. Ulijn, Curr. Opin. Biotechnol. 2010, 21, 401-411 ; R. J. Williams, A. M. Smith, R. Collins, N. Hodson, A. K. Das, R. V. Ulijn, Nat. Nano. 2009, 4, 19-24). These two works rest on the use of a protease (thermolysin) to catalyze the condensation and hydrolysis of the peptide bonds. Although of great interest, the addition of proteases in a DCL has limitations due to their substrate specificity, making the incorporation of non-natural amino acids as well as the development of isoenergetic libraries challenging. Their use is also not compatible with peptide-containing targets which could be damaged by the catalyst itself.

Therefore, and despite its manifest potential towards several domains of research, the design of an abiotic (i.e. enzyme free) synthetic approach to perform dynamic covalent formation, disruption, and exchange of peptide bonds on a practical time scale and in mild conditions is unprecedented.

### Aims of the invention

The invention aims to provide a reversible amide bond or peptide junction.

The invention aims to provide dynamic molecules.

The invention aims to provide combinatorial libraries of molecules, especially dynamic combinatorial libraries.

More particularly, the invention aims to provide combinatorial libraries of peptides, especially dynamic combinatorial libraries of dynamic peptides.

The invention aims to provide a reversible chemical reaction for preparing dynamic molecules. More particularly the invention aims to provide a reversible chemical reaction for preparing dynamic peptides under physiological conditions.

The invention aims also to provide a method for the discovery of therapeutically active ingredients.

### Description of the Invention

The invention relates to a new methodology based on Native Chemical Ligation (NCL) to prepare dynamic peptides which may be performed at neutral pH with fast exchange rates.

NCL is one of the most popular tools for the synthesis of large peptides and small proteins. The power of this non-reversible reaction resides in the straightforward formation of a native peptide bond from a C-terminal thioester and a N-terminal cysteine under physiological conditions. Interestingly, and although NCL is not a chemical reaction involved in any biochemical process, a mechanism similar to a reverse NCL reaction (retro-NCL) is found in the self-splicing of inteins (G. Volkmann, H. Mootz, Cell. Mol. Life Sci. 2013, 70, 1185-1206 *;* N. H. Shah, T. W. Muir, Isr. J. Chem. 2011, 51, 854-861). These proteins fold in a catalytically active state in which the first step of the retro-NCL, the N→S acyl shift of an internal cysteine residue, is favored. It has been proposed that in the folded intein, the scissile bond at the cysteine residue is preferentially in a *cis* or distorted conformation favoring the intramolecular nucleophilic attack of the cysteine thiol (a) G. Volkmann, H. Mootz, Cell. Mol. Life Sci. 2013, 70, 1185-1206 ; J. Binschik, H. D. Mootz, Angew. Chem. Int. Ed. 2013, 52, 4260-4264) (with an equibrium constant 4000 times larger than that of the corresponding *trans* conformation) (H. Paulus, Annu. Rev. Biochem. 2000, 69, 447-496). The resulting activated thioester is then able to undergo transthioesterification and subsequent S→N acyl transfer to irreversibly yield a novel truncated protein. Such a biological process reveals the intrinsic reversibility of the NCL reaction when peptides containing cysteine residues are placed under the appropriate catalytic environment. This observation sparked the interest of the present inventors for the development of a novel methodology extending NCL to dynamic covalent chemistry. The inventors succeeded by using simple chemical modifications of cysteine residues to promote the retro-NCL, and in particular the N→S acyl shift under neutral aqueous conditions.

In the literature, peptide thioester subtrates for the NCL have been already prepared by fragmentation of C-terminated cysteine peptides using the retro-NCL, but under harsh acidic conditions and in the presence of a large excess of an aliphatic thiol (J. Kang, J. P. Richardson, D. Macmillan, Chem. Commun. 2009, 407-409 ; Masania, J. Li, S. J. Smerdon, D. Macmillan, Org. Biomol. Chem. 2010, 8, 5113-5119 ; J. P. Richardson, C.-H. Chan, J. Blanc, M. Saadi, D. Macmillan, Org. Biomol. Chem. 2010, 8, 1351-1360 ; J. Kang, N. L. Reynolds, C. Tyrrell, J. R. Dorin, D. Macmillan, Org. Biomol. Chem. 2009, 7, 4918-4923). The resulting aliphatic thioester can be isolated from uncharacterized impurities, and used subsequently in a NCL with another N-terminal cysteine-peptide. This methodology is also limited to C-terminal cysteine peptides as it was demonstrated that internal ones are too stable. Furthermore, these conditions would not be compatible with the "forward" NCL that is most efficiently performed at neutral pH and room temperature. Very recently, peptides containing a C-terminal bis(2-sulfanylethyl)amino group were shown independently by the groups of Melnyk and Liu (N. Ollivier, J. Dheur, R. Mhidia, A. Blanpain, O. Melnyk, Org. Lett. 2010, 12, 5238-5241 ; W. Hou, X. Zhang, F. Li, C.-F. Liu, Org. Lett. 2011, 13, 386-389) to undergo N→S acyl shift under neutral (pH 7 and 37ºC) to mildly acidic (pH 4 to 6) aqueous conditions. The unprecedented reactivity of this tertiary amide was explained by the fact that in bis(2-sulfanylethyl)amino derivatives, the need for *cis-trans* isomerization prior to the N→S acyl shift is obviated as there is always one thiol group correctly positioned for the intramolecular thiolysis reaction to occur. In these studies, the activated thioesters obtained were subsequently submitted to an irreversible NCL with N-terminal cysteines.

Based on this knowledge, and as a first attempt to develop a fully dynamic NCL, the present inventors decided to replace internal and N-terminal cysteine residues used in NCL by a non-natural "dynamic amino acid" containing a thiol function. This attempt was successful. The inventors then generalized this concept to other molecules notably to prepare a dynamic combinatorial library, screen therapeutically active ingredients, and prepare new dynamic molecules and materials.

Accordingly the invention relates to a mixture of molecules comprising at least molecules (p1p2) as defined by formula (I) and at least molecules (p3) as defined by formula (II) 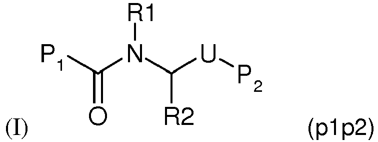 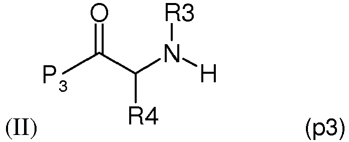
wherein R1, R2, R3, R4, P1, P2, and P3 are each independently atomic groups;
wherein at least one of R1 and R2 is -CH2(CH2)ₓ-SH wherein x is 0, or 1 to 6;
wherein at least one of R3 and R4 is -CH2(CH2)ₓ-SH wherein x is 0, or 1 to 6;
wherein -U- is an aliphatic group optionally substituted, for example -C(RaRb)-, wherein Ra and Rb are atomic groups, or an aromatic group optionally substituted, or -C(O)-, -O-, - S-, or a phosphorus containing function.
Such a phosphorus containing function is for example :-P(O)(Ra)- or -O-P(O)(Ra)-, - P(O)(ORa)-, -O-P(O)(ORa)-, -O-P(O)(Ra)-O-, or O-P(O)(ORa)-O-.
U and R2 may form together a ring such as a cycloaliphatic group (including cycloalkanes, cycloalkenes groups) or an aromatic group (aryl, including phenyl rings).

The invention also relates to molecules of this mixture.

In one embodiment 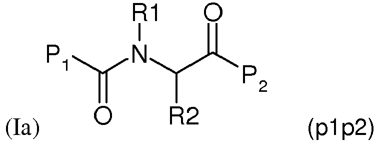 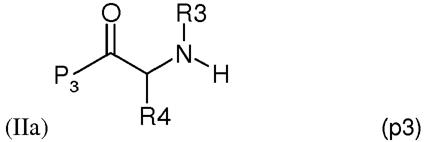
wherein R1, R2, R3, R4, P1, P2, and P3 are each independently atomic groups,
wherein at least one of R1 and R2 is -CH2(CH2)ₓ-SH wherein x is 0, or 1 to 6,
wherein at least one of R3 and R4 is -CH2(CH2)ₓ-SH wherein x is 0, or 1 to 6. In this specific embodiment U is carbonyl (-C(O)-).

In one embodiment, "x" is from 1 to 4.

In one embodiment, for R2 and R4, "x" is 0 and for R1 and R3, "x" is 1.

More particularly, said mixture further comprises at least molecules (p1p3) as defined by formula (III) and at least molecules (p2) as defined by formula (IV) 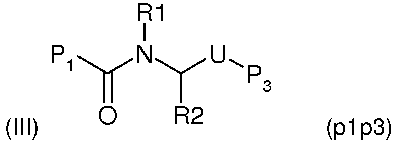 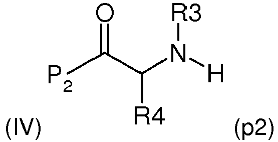 wherein R1, R2, R3, R4, P1, P2, and P3 are as defined in the invention.

In one embodiment U is carbonyl.

Atomic group P1, P2 and P3 may be exchanged between original molecules, providing different molecules in the mixture. This allows preparing a dynamic mixture of molecules.

The term "dynamic" notably refers in this context to the presence of a reversible junction or chemical linkage between molecules P1, P2 and P3 so atomic groups P1, P2, P3 may be exchanged between each other, for example by any combination of N->S acyl shift, transthioesterification and S->N acyl shift. In one embodiment, molecule of formula (I) and molecule of formula (II) will reorganize each other by exchanging atomic groups P1, P2 and/or P3.

The mixture of molecules may comprise different molecules of structures (I), (II), (III) and/or (IV).

The invention relates to a mixture as defined according to the invention, said mixture comprising the following chemical reaction or equilibrium: 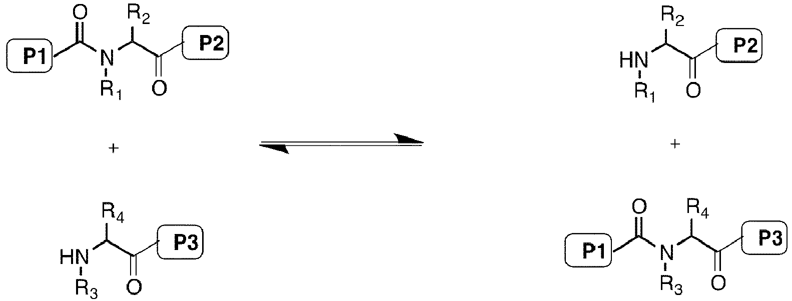 wherein the equilibrium between the concentrations of compounds (p1p2), (p3), (p1 p3) and (p2) is dependent on external conditions. By changing the external conditions, the equilibrium of this reaction may change and therefore the concentrations of compounds (p1p2), (p3), (p1p3) and (p2).

For example a mixture of molecules according to the invention may comprise "n" different atomic groups P1, P2, P3, ..., Pn, wherein "n" represents the number of different structures of atomic groups which may be exchanged between molecules of formula (I), (II), (III) or (IV) present in the mixture.

In one embodiment, "n" represents a number above 5, preferably above 10, more preferably above 100, for example above 1000, or above 10³, or above 10⁴ or above 10⁵,

The invention relates to a mixture of molecules, wherein P1, P2, and P3 are each independently selected from the group consisting of single unit molecules (monomers), for example therapeutic molecules, peptides, nucleotides, and polymers molecules, for example oligopeptides, polypeptides, oligonucleotides, polynucleotides, polyamides, including non-peptide polyamides, including aliphatic polyamides and aromatic polyamides.

According to one embodiment P1, P2, P3 are each independently molecules comprising amino acid and/or amide bonds, for example P1, P2, P3 are each polymers containing amino acid bonds. Such molecules containing amino acid bonds are for example selected from the group consisting of peptides, oligopeptides, oligopeptides analogs, polypeptide or polypeptide analogs, proteins, or proteins analogs, nucleotides, oligonucleotides, polynucleotides.

The term "polypeptide" is notably refering to an amino acid sequence, including to products of post-expression modifications of a polypeptide, including without limitation glycosylation, acetylation, phosphorylation, amidation and derivatization. A polypeptide may be derived from a natural biological source or produced by synthesis, including recombinant technology. It may be prepared by full or partial synthesis or translated from a designated nucleic acid sequence. It may be generated in any manner, including by chemical synthesis. A polypeptide of the invention may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. The terms "polypeptide analog" include any molecule which includes at least one polypeptide sequence.

The term "polynucleotide" refers notably to a nucleic acid sequence, and refers notably to a nucleic acid molecule or construct, for example DNA, cDNA, RNA, messenger RNA (mRNA) or plasmid DNA (pDNA). A polynucleotide may comprise a conventional phosphodiester bond or a non-conventional bond (e.g., an amide bond, such as found in peptide nucleic acids (PNA)).

In one specific embodiment, P1, P2, and P3 are each independently selected from the group consisting of PA 6, PA 12, PA 11, PA 4/6 , PA 6/6, PA 6/9, PA 6/10, PA 6/12, PA 10/10, PA 10/12, PA 66, and copolymers thereof, for example PA6/66 and PA 66/610; Polyphthalamides, for example PA 6T (hexamethylenediamine + terephthalic acid); aramides (aromatic polyamides), aliphatic-containing polyamides, alkyl-containing polyamides, aliphaticaryl-containing polyamides, alkylaryl-contanining polyamides, for example paraphenylenediamine + terephthalic acid, p-phenylene terephthalamides (PPTA), poly-metaphenylene isophthalamides (MPIA), oligonucleotides, polynucleotides, DNA, RNA, and Peptide nucleic acid (PNA).

In one specific embodiment, P1, P2, and P3 are each independently selected from the group consisting of peptides, oligopeptides, and polypeptides.

R1, R2, R3, R4, are each independently selected from the group consisting of H, aliphatic groups, including alkyls, alkyls containing a thiol function, aryls, alkylaryls, alkylaryls containing a thiol function (-SH), wherein at least one of R1 and R2 is - CH2(CH2)x-SH wherein x is 0, or from 1 to 6, and wherein at least one of R3 and R4 is - CH2(CH2)x-SH wherein x is 0, or from 1 to 6.

Ra and Rb are not particularly limited and may be for example each independently selected from the group consisting of H, aliphatic groups, including, hydrocarbyls, optionally substituted, heterohydrocarbyls, alkyls, optionally substituted, heteroalkyls, alkenyls, optionally substituted, heteroalkenyls, alkynyls, optionally substituted, heteroalkynyls, alkoxys, aryloxys, hydroxyls, aryls, substituted aryls, alkylaryls,heteroaryls, aralkyls, aralkylenes, alkaryls, alkarylenes, halogen atoms (Cl, F, I or B), haloalkyls, haloalkenyls, haloalkynyls, heteroalkyls, heterocycles, heteroaryls, heteroatom-containing groups, aminos, amines, cycloalkyls, acyls, aroyls, dialkylamines, alkylamidos, alkoxycarbonyls, aryloxycarbonyls, carbomoyls, alkyl- and dialkyl-carbamoyls, acyloxys, acylaminos, aroylaminos, and any combination thereof.

In one embodiment, at least one of R1 and R2, or both, are -CH2(CH2)x-SH wherein x is independently selected from 1 to 6, and wherein at least one of R3 and R4, or both, are -CH2(CH2)x-SH wherein x is independently selected from 1 to 6.

In an advantageous embodiment, R1 and R3 are -CH2(CH2)ₓ-SH wherein x is from 0 to 6, and R2 and R4 are -H, an aliphatic, aromatic or amino acid side chain, or CH2(CH2)ₓ-SH where x is from 0 to 4.

In one embodiment, R2 and R4 are -CH2-SH.

In one embodiment R1 and R4 are -CH2-CH2-SH.

In an advantageous embodiment, R₁ is an -H, or an aliphatic group, preferably selected from -CH₃, -CH₂CH₃, -CH(CH₃), and -C(CH₃)₃, and R₂ is -CH2(CH2)ₓ-SH where x is from 0 to 4.

R1, R2, R3 and R4 may represent same or different atomic groups.

Advantageously -NR1-CR2-C(O)- represents a N-(alkyl)-cysteine, typically a N-(methyl)-cysteine.

Advantageously, -NR3-CR4-C(O)- represents a N-(alkyl)-cysteine, typically N-(methyl)-cysteine.

Preferably, alkyls are linear or branched C1-C6 alkyls, more preferably C1, C2, C3, C4-alkyls.

Preferably, aryls are 5 to 10 members aromatic rings, preferably C5-C10 aromatic rings, more preferably C5 or C6 aromatic rings, such as furanyl and phenyl.

Preferably, alkylaryls are alkyls substituted by a 5 to 10 members aromatic rings, preferably C5-C10 aromatic rings, more preferably C5 or C6 aromatic rings, such as furanyl and phenyl.

Preferably, alkylaryls containing a thiol function are for example phenyl or benzyl bearing a thiol function -CH2(CH2)x-SH wherein x is 0, or 1 to 6.

In one embodiment, the atomic group among R1 and R2 which is not -CH2(CH2)x-SH is selected from the group consisting of H, alkyl, preferably C1-6, for example C1, C2, C3, C4, C5 or C6, more preferably are H or methyl.

In one embodiment, the atomic group among R3 and R4 which is not -CH2(CH2)x-SH is selected from the group consisting of H, alkyl, preferably C1-6, for example C1, C2, C3, C4, C5 or C6, more preferably are H or methyl.

In one specific embodiment, R1, R2, R3, R4, are each -CH2-SH or -CH2-CH2-SH, preferably -CH2-SH.

According to one embodiment, R1 and R2 are -CH2(CH2)ₓ-SH, and/or wherein R3 and R4 are -CH2(CH2)ₓ-SH.

According to one embodiment, molecules (p1p3) and (p3) are of formula (Ib) and (IIb) as follows:

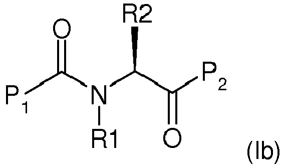

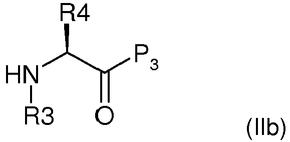

According to one embodiment, R1 is H, aliphatic, including alkyl, preferably C1-6-alkyl, for example C1, C2, C3, C4, C5 or C6-alkyl, more preferably is H or methyl.

According to one embodiment, R3 is H, aliphatic, including alkyl, preferably C1-6-alkyl, for example C1, C2, C3, C4, C5 or C6-alkyl, more preferably is H or methyl.

According to one embodiment, R1 is -CH2(CH2)x-SH wherein x is 0, or 1 to 6 and R2 is an amino acid side chain.

According to one particular embodiment, molecule (p1p2) is of following formula (Ic): 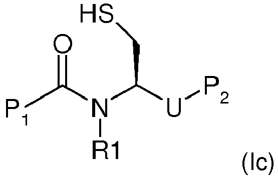
wherein R1 is as defined in the present invention, and preferably is -H, -CH2(CH2)x-SH wherein x is 0, or 1 to 6, preferably -CH2-CH2-SH, or an aliphatic group, preferably selected from -CH₃, -CH₂CH₃, -CH(CH₃), and -C(CH₃)₃,
wherein -U- is an aliphatic group optionally substituted, for example -C(RaRb)-, wherein Ra and Rb are atomic groups, or an aromatic group optionally substituted, or -C(O)-, -O-, - S-, or a phosphorus containing function;

Preferably, in this last embodiment, the mixture comprises a molecule (p3) of the following formula : 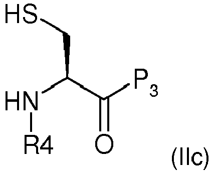 wherein R4 is as defined in the present invention, and preferably is -H, -CH2(CH2)x-SH wherein x is 0, or 1 to 6, preferably -CH2-CH2-SH, or an aliphatic group, preferably selected from -CH₃, -CH₂CH₃, -CH(CH₃), and -C(CH₃)₃.

According to one particular embodiment, molecule (p1p2) is of following formula (Id): 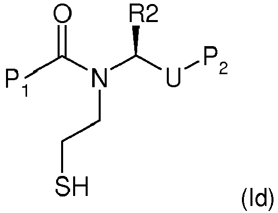 wherein R2 is as defined in the present invention, and preferably is -H, -CH2(CH2)x-SH wherein x is 0, or 1 to 6, preferably -CH2-SH, or is an amino acid side chain wherein-U- is an aliphatic group optionally substituted, for example -C(RaRb)-, wherein Ra and Rb are atomic groups, or an aromatic group optionally substituted, or -C(O)-, -O-, -S-, or a phosphorus containing function.

In one embodiment, in general and more specifically in formula (I), (Ia), (Ib), (Ic) or (Id) or (II), (IIa), (IIb), (IIc) or (IId), U is carbonyl (-C(O)-). In one embodiment U is - CH2(CH2)x-, wherein x is from 0 to 6, for example, 0, 1, 2, 4, 5, or 6.

Preferably, in this last embodiment, the mixture comprises a molecule (p3) of the following formula : 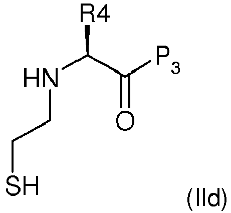 wherein R4 is as defined in the present invention, and preferably is an amino acid side chain.

In the present invention, amino acids are composed of amine (-NH2) and carboxylic acid (-COOH) functional groups, along with a side-chain specific to each amino acid. Structurally they can be classified according to the functional groups locations as alpha-(α-), beta- (β-), gamma- (γ-) or delta- (δ-) amino acids; other categories relate to polarity, pH level, and side chain group type (aliphatic, acyclic, aromatic, containing hydroxyl or sulfur, etc.).

| **Name** | **Symbol** | **Side Chain (R2) is for example** |
|---|---|---|
| Alanine | A, Ala | CH₃- |
| Arginine | R, Arg | HN=C(NH₂)-NH-(CH₂)₃- |
| Asparagine | N, Asn | H₂N-CO-CH₂- |
| Aspartic acid | D, Asp | HOOC-CH₂- |
| Cysteine | C, Cys | HS-CH₂- |
| Glutamine | Q, Gln | H₂N-CO-(CH₂)₂- |
| Glutamic acid | E, Glu | HOOC-(CH₂)₂- |
| Glycine | G, Gly | H- |
| Histidine | H, His | 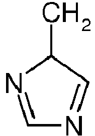 |
| | | 3H-imidazol-4-yl |
| Isoleucine | I, Ile | CH₃CH₂CH(CH₃)- |
| Leucine | L, Leu | (CH₃)₂-CH-CH₂- |
| Lysine | K, Lys | H₂N-(CH₂)₄- |
| Methionine | M, Met | CH₃-S-(CH₂)₂- |
| Phenylalanine | F, Phe | Phenyl-CH₂- |
| Proline | P, Pro | 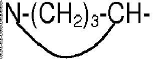 |
| | | (cyclic) |
| Serine | S, Ser | HO-CH₂- |
| Threonine | T, Thr | CH₃-CH(OH)- |
| Tryptophan | W, Trp | 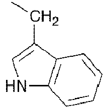 |
| | | 1H-indol-3-yl-CH₂- |
| Tyrosine | Y, Tyr | 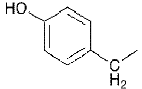 |
| | | 4-OH-Phenyl-CH₂- |
| Valine | V, Val | (CH₃)₂CH- |

In one embodiment, amino acid sides chains R2 is such that NR1-CR2-C(O)-represents an amino acid.

In one embodiment, amino acid sides chains R4 is such that NR4-CR3-C(O)-represents an amino acid.

Natural amino acids are typically: Histidine, Alanine, Isoleucine, Arginine, Leucine, Asparagine, Lysine, Aspartic acid, Methionine, Cysteine, Phenylalanine, Glutamic acid, Threonine, Glutamine, Tryptophan, Glycine, Valine, Ornithine, Proline, Selenocysteine, Serine, and Tyrosine.

Non-standard amino acids that are found in proteins are formed for example by chemical synthesis or by post-translational modification, which is modification after translation during protein synthesis. Some nonstandard amino acids are not found in proteins.

In one embodiment said amino acid is selected from Gly, Cys, His or any other natural or unnatural aminoacid.

In one embodiment, in general and more specifically in formula (I), (Ia), (Ib), (Ic) or (Id) or (II), (IIa), (IIb), (IIc) or (IId), R2 is -H (N(2-thioethyl)glycine). Preferably, a mixture according to the invention comprises terminal N(2-thioethyl)glycine, for example a molecule of formula (IId) wherein R4 is glycine side chain or any other amino acid side chain. R2 and R4 may represent the same or different atomic groups.

In one embodiment, in general and more specifically in formula (I), (Ia), (Ib), (Ic) or (Id) or (II), (IIa), (IIb), (IIc) or (IId), R2 is CH₃ (N(2-thioethyl)alanine). Preferably, a mixture according to the invention comprises terminal N(2-thioethyl) alanine, for example a molecule of formula (IId) wherein R4 is alanine side chain or any other amino acid side chain.

According to one embodiment said mixture comprises more than "n" different molecules (I1) to (In) of formula (I) wherein n represent a number above 5, preferably above 10, more preferably above 100, for example above 1000, or above 10³, or above 10⁴ or above 10⁵, wherein P1 and/or P2 represent different atomic groups when comparing each different molecules (I1) to (In), thereby defining a specific structure for each of molecules (I1) to (In).

In other words, P1 and/or P2 are not identical between different molecules (I1) to (In). P1 and P2 of molecule (I1) may be defined as P1/1 and P2/1, P1 and P2 of molecule (In) may be defined as P1/n and P2/n. Alternatively, molecule (I1) may be defined as comprising the sequence P1/1-P2/1, molecule (In) may be defined as comprising the sequence P1/n-P2/n wherein the sequences P1/1-P2/1 and P1/n-P2/n represent different chemical structures.

According to one embodiment said mixture comprises more than "n" different molecules (II1) to (IIn) of formula (II) wherein n represent a number above 5, preferably above 10, more preferably above 100, for example above 1000, or above 10³, or above 10⁴ or above 10⁵, wherein P3 represents different atomic groups when comparing each different molecules (II1) to (IIn), thereby defining a specific structure for each of molecules (II1) to (IIn).

In other words, P3 are not identical between different molecules (II1) to (IIn). P3 of molecule (II1) may be defined as P3/1, P3 of molecule (IIn) may be defined as P3/n. Alternatively, molecule (II1) may be defined as comprising the atomic group P3/1, molecule (In) may be defined as comprising the atomic group P3/n wherein the atomic groups P3/1 and P3/n represent different chemical structures.

For example, a mixture comprising at least 5 different structures of molecules of formula (I) may be referred to as I1, 12, 13, 14, and I5 each having a different structure by incorporating different atomic structures or sequences P1/1-P2/1, P1/2-P2/2, P1/3-P2/3, P1/4-P2/4, P1/5-P2/5.

The invention relates to a combinatorial library of molecules comprising a mixture as defined according to the invention.

The term "combinatorial library" is defined herein to mean a library of molecules containing a large number, typically between 10 and 10⁶ different compounds typically characterized by different sequences of atoms or in other words different atomic groups, here defined as P1, P2, and P3, or a combination of different atomic groups.

Combinatorial synthesis consists in deliberate construction of a set of molecules based on a specific design of chemical reactions that lead to linking selected atomic groups or monomers in various combinations. Thus obtained library is then searched in order to identify elements having desired properties. As a result of the selection process, active molecules may be isolated from the library.

An important objective of combinatory chemistry is to generate a large number of novel compounds that can be screened to generate lead compounds for research, including pharmaceutical and polymer technologies.

Theoretically the total number of compounds which may be produced for a given library is limited only by the number of reagents available to form substituents on the variable positions on the library's molecular scaffold. The combinatorial process is a powerful tool, both in the generation of compounds and in their screening, thereby greatly enhancing the opportunity and efficiency of active molecules discovery, notably new drugs.

The invention more specifically relates to a "dynamic combinatorial library" or "dynamic mixture". The terms "combinatorial library" or "dynamic mixture" are defined herein to mean a combinatorial library or a mixture which is dynamic in the sense that molecules of the combinatorial library or mixture may reorganize between each other by exchanging specific atomic groups, such as for examples atomic groups P1, P2 and P3. Notably such reorganization may occur when one or more external conditions change, for example such as changing one or more physical parameters, such as for example temperature, pressure, pH, electric current, magnetic or electromagnetic radiation, etc, and/or one or more chemical parameters, such as for example adding or changing solvent of the library or mixture, adding chemical additives to the library or mixture, and/or adding one or more potentially active molecules to the library or mixture. "Potentially active molecules" means molecules that may present an activity of interest and which are screened through its interaction with the molecules of the library. Such potentially active molecules are typically drugs or therapeutically or biologically active molecules. Such potentially active molecules may also be dynamic polymers. "Dynamic polymers" polymers present specific properties which change upon external stimuli, such as for example changing temperature, pressure, pH, electric current, magnetic or electromagnetic radiation, etc, and/or in the presence of one or more chemical parameters, such as for example adding or changing solvent of said polymer, adding chemical additives to said polymer, and/or putting said polymer into contact with other molecules.

For example, a dynamic combinatorial library comprising at least 5 different structures of molecules of formula (I) which may be referred to as I1; 12; 13; 14; and I5 each having a different structure by incorporating different atomic structures or sequences P1/1-P2/1, P1/2-P2/2, P1/3-P2/3, P1/4-P2/4, P1/5-P2/5. Upon change of conditions, typically upon putting said molecules of formula (I) into contact with one or more potentially therapeutic active molecules, said dynamic combinatorial library molecules of formula (I) may reorganize into a different set of molecules of formula (I) thereby giving rise to different atomic structures or sequences, for example : P1/1-P2/5, P1/2-P2/1, P1/3-P2/4, P1/4- P2/2, P1/5-P2/3, etc. (without any limitation on the possible reorganizations).

It should be noted here that atomic groups defined as P1, P2, and P3 may give rise to new atomic groups P4, P5; P6, ..., Pn, wherein n is the number of different atomic groups. Such new atomic groups P4, P5; P6, ..., Pn, may be defined here as rearranged atomic groups.

In one preferred embodiment, said dynamic library may exchange atomic groups defined as P1, P2, and P3 or produce rearranged atomic groups by combination of N->S acyl shift, transthioesterification and S->N acyl shift between at least molecules of formula (I) and (II) present.

More particularly the invention relates to a method for preparing a compound (P1 P2) as defined according to the invention, said method comprising the following chemical reaction: 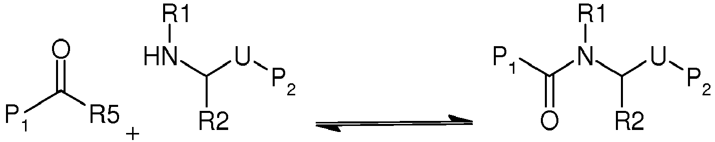
wherein R1, R2, P1, and P2 are each independently atomic groups,
wherein at least one of R1 and R2 is -CH2(CH2)ₓ-SH wherein x is 0, or 1 to 6,
wherein -U- is an aliphatic group optionally substituted, for example -C(RaRb)-, wherein Ra and Rb are atomic groups, or an aromatic group optionally substituted, or -C(O)-, -O-, - S-, or a phosphorus containing function;
wherein R5 is a thioester -SR6 with R6 is a hydrogen atom or an aliphatic or aromatic group, or an amine having the chemical structure below to form an amide together with-C(O)P1: 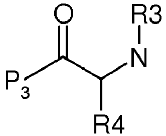
wherein R3, and R4 are each independently atomic groups,
wherein at least one of R3 and R4 is -CH2(CH2)ₓ-SH wherein x is 0, or 1 to 6.
In one embodiment, said method comprising the following chemical reaction: 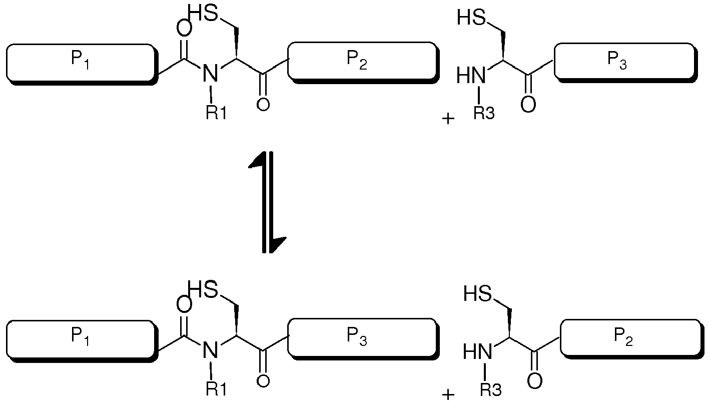 wherein R1, R2, R3, R4, P1, P2, and P3 are as defined according to the invention.
In one embodiment, said method comprising the following chemical reaction: 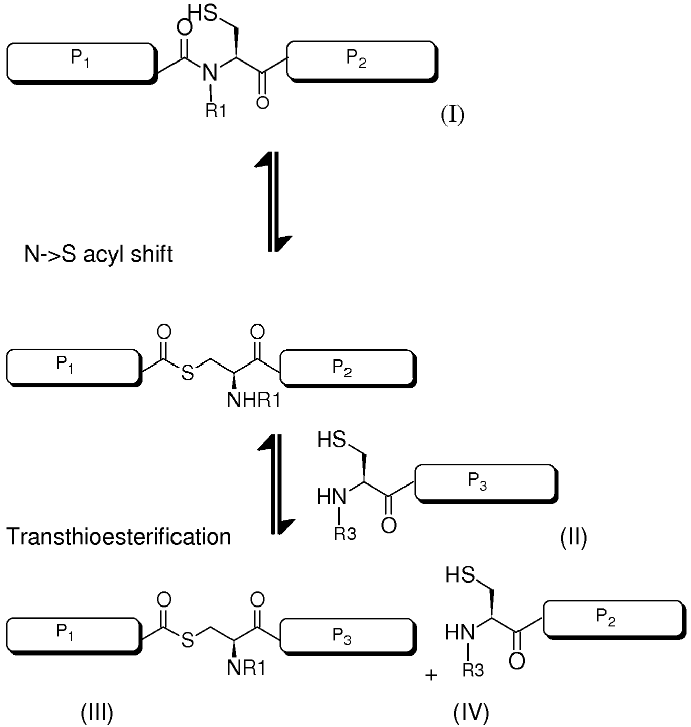 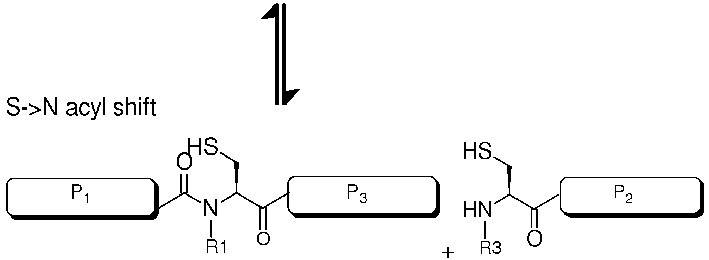
wherein P1, P2, P3 are polymers,
wherein R1 and R3 are each independently H, alkyl, preferably C1-6-alkyl, for example C1, C2, C3, C4, C5 or C6-alkyl, more preferably is H or methyl, or -CH2(CH2)ₓ-SH
wherein x is 0, or 1 to 6.

In a particular embodiment, said library comprises molecules presenting internal and/or terminal cysteines. "Internal" means that cysteine is inside a molecule and is opposite to "external" which means that cysteine is present at at least one extremity of a molecule. In one embodiment said library present molecules presenting internal and terminal cysteines. In one embodiment said library present molecules presenting internal cysteines and molecules presenting terminal cysteines.

Preferably said chemical reaction (claim 12) is performed in aqueous buffer at pH 6-9. Preferably said chemical reaction (claim 12) is performed in the presence of a reducing or "deprotecting" agent for thiolated molecules, typically dithiothreitol (DTT). Preferably said agent is in aqueous buffer at pH 6-9.

Preferably said chemical reaction (claim 12) is performed at a temperature between 15 and 50°C, preferably between 20 and 45°C. Preferably said chemical reaction (claim 12) is performed at a temperature between 32 and 42°C, typically at about 37"°C.
The invention also relates to a molecule comprising or having the chemical structure below: 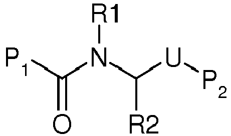
wherein -U- is an aliphatic group optionally substituted, for example -C(RaRb)-, wherein Ra and Rb are atomic groups, or an aromatic group optionally substituted, or - C(O)-, -O-, -S-, or a phosphorus containing function;
wherein R1 is -CH2(CH2)x-SH wherein x is 0, or 1 to 6, preferably -CH2-CH2-SH; or H or an aliphatic group, preferably selected from -CH₃, -CH₂CH₃, -CH(CH₃), and -C(CH₃)₃ ;
wherein R2 is -H, -CH2(CH2)x-SH wherein x is 0, or 1 to 6, preferably -CH2-SH, or is H or an amino acid side chain;
wherein at least one of R1 and R2 is -CH2(CH2)ₓ-SH wherein x is 0, or 1 to 6, and
wherein P1 and P2 are as defined according to the invention.
Said molecule comprises or has the chemical structure below: 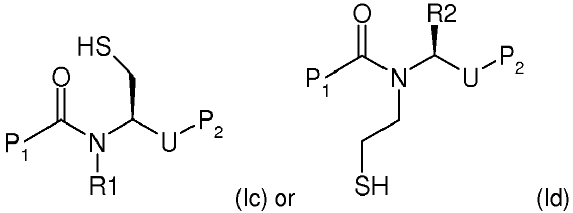 as defined in the present invention.

In one embodiment -U- is - (CH2)n-, wherein n is from 0 to 6 In one embodiment -U-is -CH2(CH2)n-, wherein n is 0, 1, 2, 3, 4, 5 or 6.

In one embodiment -U- is phenyl.

In one specific embodiment, said molecule is a polymer. Polymers include homopolymers, copolymers, including diblocks-, triblocks-, multi-blocks copolymers. More particularly, said molecule is a dynamic polymers, for example polyamide dynamic polymers. Said polymer includes a side chain or pendant chain including an internal thio function, preferably represented by -CH2(CH2)ₓ-SH wherein x is 0, or 1 to 6, preferably x is 0 or 1.

In one embodiment, R1 brings advantageously a dynamic character to said molecule. In other words, without R1 the molecule does not present the same dynamic properties.

For example, the invention relates to dynamic polymers analogs of Kevlar incorporating a dynamic N-(2-thioethyl)-amide junction to reversibly connect Kevlar oligomers. Such dynamic polymers analogs may present the following structure:

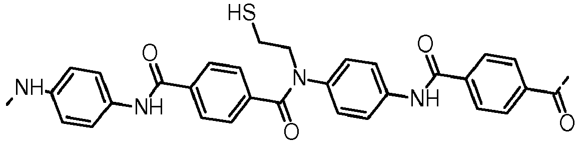

In such an example, U and R2 form together a phenyl ring.

For example, the invention relates to dynamic polymer analogs of Nylon incorporating a dynamic N-(2-thioethyl)-amide amide junction to reversibly connect Nylon oligomers. Such dynamic polymers analogs may present the following structure:

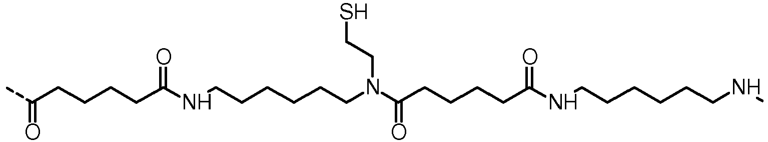

In one embodiment, said molecule comprises a therapeutically or biologically active ingredient, in other words a drug or a prodrug. In said molecule P1, and/or P2 may represent such a drug or prodrug. A prodrug is a molecule or atomic group which, in vivo, undergoes a chemical modification of its chemical structure (including change in the spatial structure of a tridimensional molecule), for example by cleavage of a bond or function, for example an ester or amide bond or equivalent bonds or functions, to provide the drug, preferably near the targeted site of therapeutic action.

In one specific embodiment, said molecule is a polypeptide comprising a polypeptide drug or polypeptide prodrug.

Such molecule may undergo a chemical modification upon contact with a molecule of formula (II) or may undergo in vivo modification so as to deliver molecules P1 and/or P2 as drug or prodrug.

During inventor's investigations of the exchange reactions between peptide fragments by reversible NCL, in the presence of an excess of DTT (25mM), it was observed the formation of peptides thioesters formed by the residual cleavage of the peptides incorporating N(methyl)- or N(2(thioethyl)-cysteine by transthioesterification with DTT. In addition, a small proportion of hydrolysed peptide resulting from the hydrolysis of this thioester under basic aqueous conditions was also observed. These two observations suggest that peptides incorporating for example N-(Methyl)- or N(2-thioethyl)cysteine are prone to cleavage by aqueous free thiols. As reversible covalent bonds have been used for the controlled release of therapeutic agents in vivo and in vitro, this controlled peptide cleavage could be applied to the delivery of peptidic therapeutic agents. The invention also relates to a method for in vivo controlled peptidic drug release, notably upon exposure to intracellular levels of gluthathione.

The invention also relates to a method for delivering a drug or prodrug to a subject in need thereof, preferably a human, wherein said method comprises administering a compound of formula (I).

The invention also relates to peptide nucleic acid (PNA) analogs incorporating dynamic amide junctions to reversibly connect the PNA building blocks. Such PNA building blocks may present the following structures:

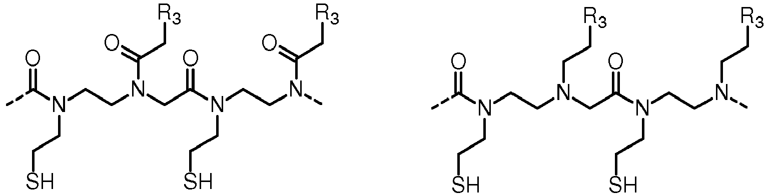

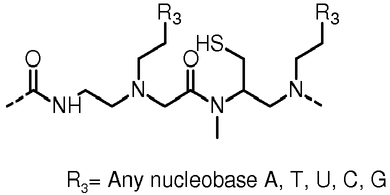

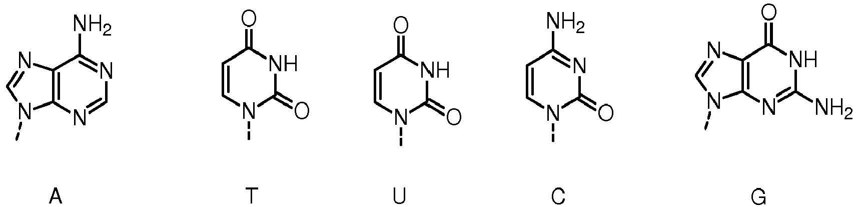

The present invention relates also to a method for preparing such molecules and mixtures of molecules.

In one embodiment, said method comprises incorporating a fragment -NR1-CR2-CO- of formula (I) as defined in the present invention into a molecule. Said molecule may present acid and amine terminal functions. Typically said fragment -NR1-CR2-CO- may be introduced or incorporated into a polymer presenting or comprising the P1-CO-P2 structure, wherein P1-CO present a C-terminal end (COOH terminus) and P2 present a N-terminal end (NH2 terminus), to give the sequence P1-CO-NR1-CR2-CO-P2.

In one specific embodiment, said fragment -NR1-CR2-CO- of formula (I) as defined in the present invention is incorporated into a polymer comprising amide functions, preferably a polyamide, including an oligopeptide, a polypeptide, peptides, oligopeptides, oligopeptides analogs, polypeptide or polypeptide analogs, proteins, or proteins analogs, oligonucleotides, oligonucleotides analogs, polynucleotides, polynucleotides analogs, polyamides, including non-peptide polyamides, including aliphatic polyamides and aromatic polyamides.

In one embodiment, said method comprises coupling said fragment -NR1-CR2-CO-or molecule of formula (I), or another fragment thereof, to a solid polymer, such as for example an amide resin, typically a rink amide resin. Such solid polymer is for example a solid phase for peptide synthesis to prepare peptide amides utilizing for example Fmoc-protected amino acids. The peptide sequence is typically assembled under basic or neutral conditions on said solid polymer, then the completed peptide is cleaved from the solid polymer under acid conditions.

In such an embodiment, the molecule coupled to the solid phase may be cleaved notably to isolate said molecule. Typically a peptide molecule is cleaved from a solid phase (Rink amide resin) using 10% TFA in DCM.

The molecule may be purified.

The method of the invention may also comprise an oxidizing step to oxidize molecule which are in their reduced form.

For preparing a mixture or library according to the invention , molecules of formula (I) may be dissolved or dispersed in a solvent, for example water, molecules of formula (II) may be dissolved or dispersed in a solvent, for example water. Solution or dispersion containing molecules of formula (I) and solution or dispersion containing molecules of formula (II) may be put together into contact, optionally with other solution(s) and/or dispersion(s), and/or other liquid, solid and/or gaseous or phase(s), preferably until homogeneous mixing.

In one embodiment, the invention's molecule(s) and/or invention's mixture, and/or invention's library is frozen and/or lyophilized.

Accordingly, the present invention relates to a powder of invention's molecule and/or invention's mixture, and/or invention's library.

In one embodiment, said powder of invention's molecule and/or invention's mixture, and/or invention's library is diluted or dispersed into an aqueous buffer. In one embodiment, said aqueous buffer is at pH from 6 to 9, for example 6, 7, 8, or 9 and optionally contains one or more reducing or "deprotecting" agents for thiolated molecules, typically dithiothreitol (DTT).

In one embodiment, said diluted invention's molecule, mixture, or library is analyzed for identifying molecules present. Typically analytical techniques are selected from chromatography, spectroscopy bioanalysis, radioanalytical chemistry; electrophoresis, typically gas chromatography, HPLC, UPLC, supercritical fluid chromatography, ligand binding assays, hyphenated techniques, mass spectroscopy, nuclear magnetic resonance (NMR), and any combination thereof,

In the present invention, reference to molecule of formula (I) or equivalent terms, refers to all embodiments, including specific embodiments and preferred features. This reference includes for example molecules of formula (Ia), (Ib), (Ic), etc.

In the present invention, reference to molecule of formula (II) or equivalent terms, refers to all embodiments, including specific embodiments and preferred features. This reference includes for example molecules of formula (IIa), (IIb), (IIc), etc.

### Detailed description of the invention

The invention is now described in more details by reference to a specific embodiment, without limitation. For a better understanding, the invention is described by reference to the following scheme 1 (parts (a) and (b)). In this specific embodiment, P1, P2 and P3 are amino acid sequences (notably oligopeptides or polypeptides). P1, P2 and P3 are described here as specific sequences but may be any amino acid sequence. Specific sequences were selected for purpose of illustrating and identifying the exchange according to the invention between atomic groups P1, P2 and P3.

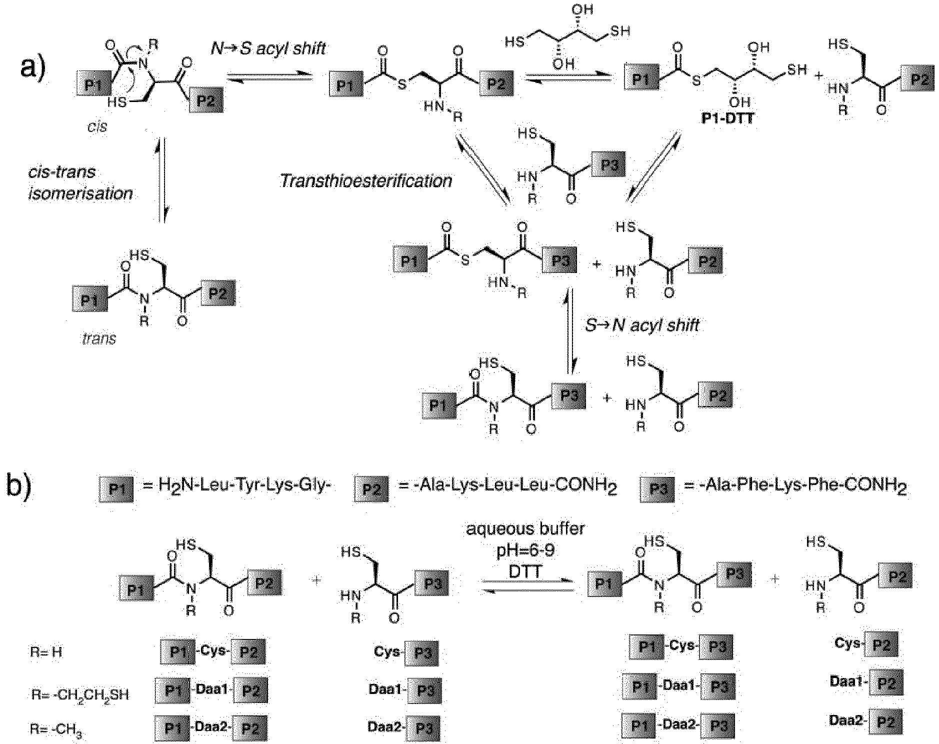

Scheme 1(a) represent a coupled equilibria describing a fully reversible NCL which involves sequences of *cis-trans* isomerisations, N→S acyl shifts, transthioesterifications, and S→N acyl shifts. Intermediate P1-DTT which is produced by the presence of dithiothreitol additive in the present study is represented for the sake of clarity.

Scheme 1(b) represent a chemical structures of the peptide fragments (P1-3) used in this study and schematic representation of a dynamic NCL based on the use of cysteine (Cys), N-(2-thioethyl)-cysteine (Daa1), and N-(methyl)-cysteine (Daa2). All peptides have a C-terminal carboxamide.

To develop a fully dynamic NCL, it was decided to replace internal and N-terminal cysteine residues used in NCL by a non-natural "dynamic amino acid" **Daa1** (scheme 1b, R=CH₂CH₂SH).

Accordingly, the invention relates to the use, combination or cooperation of peptides comprising internal and N-terminal N-(2-thioethyl)-cysteine residues. The invention further relates to a method for preparing a combinatorial library by reaction, or putting together into contact, peptides comprising internal and N-terminal N-(2-thioethyl)-cysteine residues.

In this configuration, the product of ligation after transthioesterification and S→N acyl shift still incorporates the N-(2-thioethyl)-cysteine and should be able to undergo itself a N→S acyl shift under the same conditions, making the system completely reversible and suitable for a dynamic combinatorial approach for the preparation of a dynamic combinatorial library (Scheme 1a,b).

The inventors synthesized short model peptides **P1-P3** with random sequences using conventional or microwave assisted solid phase synthesis in the Fmoc strategy (detailed synthetic protocols and full characterizations of all peptides are described in the examples). The incorporation of the 2-thioethyl unit on the N-terminal free amines of peptides immobilized on solid support was achieved using synthetic routes described in the literature (W. Hou, X. Zhang, F. Li, C.-F. Liu, Org. Lett. 2011, 13, 386-389.). For practical reasons, peptides **P1-Daa1-P2** (LYKG**Daa1**AKLL-NH₂) and **Daa1-P3** (**Daa1**AFKF-NH₂) were isolated in their oxidized form containing an intramolecular disulfide bond. Accrdongly the invention also relates to molecules comprising an intramolecular disulfide bond. These model peptides were then used to study the dynamic of the NCL by following the exchange reaction of peptides **P1-Daa1-P2** with **Daa1-P3** at pH=6 in a phosphate buffer and in presence of 0.38% w/v of dithiothreitol DTT (an additive which is added to avoid disulfide formation from thiols in the reaction mixture and which is commonly used for protein assays or even in cells). The fact that such reaction may iccur under usual assay condition is therefore very interesting and has important value.

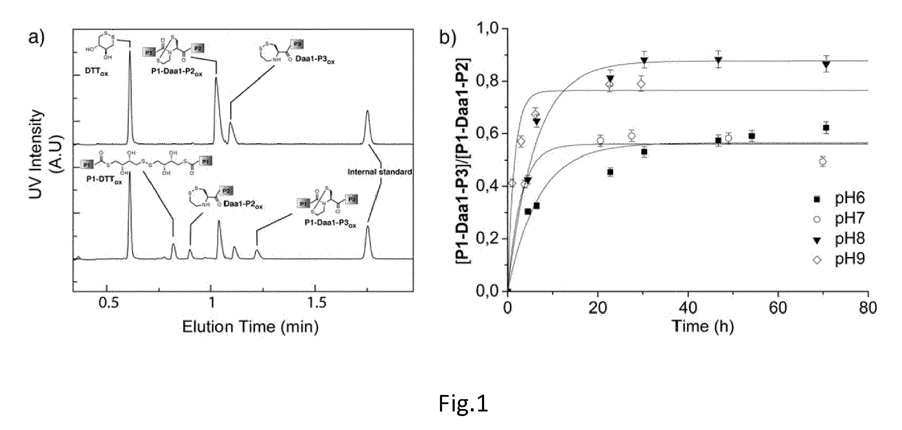

The dynamic covalent character of the NCL was unambiguously established by the appearance of peptides **Daa1-P2** and **P1-Daa1-P3** (which were also prepared independently as references, see examples). Typical UV chromatograms obtained during the course of the reaction from an Ultra Performance Liquid Chromatography coupled to Mass Spectrometry unit (Waters UPLC^{®}-MS). UPLC^{®} chromatograms of the exchange reaction between **P1-Daa1-P2** and **Daa1-P3** at pH 6 and at t=0 (*top*) and t=46 h (*bottom*) are represented on figure 1.

Importantly, the symmetric reaction consisting in the dynamic exchange starting from **P1-Daa1-P3** and **Daa1-P2** led to the same peptide distribution at equilibrium. This is in contrast with the result of the control experiment performed on peptides **P1-Cys-P2** and **Cys-P3.** At pH7, and after 4 weeks, no trace of the exchange products **P1-Cys-P3** and **Cys-P2** was detected even by mass spectrometry. Experimentally, the crude exchange mixtures were separated on UPLC^{®} HSS-T3 columns and quantitatively analyzed only after the full oxidation of all peptides in their corresponding intramolecular disulfides. The method consists in diluting the exchange reaction with a H₂O₂ solution containing an internal UV standard (3,5-dimethoxybenzoic acid) to account for potential errors during the sample preparation and injection process. A direct injection of the exchange mixture leads to complex chromatograms with peaks corresponding to the different peptides in their oxidized disulfide form with reduced free thiols overlapping, precluding a quantitative assessment of the products' distribution.

Tests were performed to cover a pH range going from mildly acidic to mildly basic conditions (Figure 1a). The dynamic recombination of **P1-Daa1-P2** with **Daa1-P3** in water leads to the rapid formation of the exchange products **P1-Daa1-P3** and **Daa1-P2** at all tested pH, with initial rates (*V₀*) of 0.25, 0.30, 0.40, and 0.55 mM.h⁻¹; and half-times of equilibration (t_{*1*/*2*}) of 5.5, 2, 4, and 1.5 h, at room temperature and at pH 6, 7, 8 and 9 respectively. This dependency on pH can be explained according to the literature on both consecutive individual steps that are required for the formation of exchange product **P1-Daa1-P3.** The rearrangements of the amide based bis(2-sulfanylethyl)amino units into their thioester form is favored by lowering the pH, suggesting that, in addition to the increase of the *cis* amide bond population, inhibition of the S→N acid shift by protonation of the amide nitrogen could shift the equilibrium towards the thioester. Conversely, the other expected rate limiting step of the dynamic NCL pathway, namely the transthioesterification, is accelerated by increasing the pH to mildly basic conditions. At lower pHs (pH 6 and 7) the presence of **P1-DTTₒₓ** resulting from the transthioesterification between the thioester generated by the N→S acyl shift of **P1-Daa1-P2** and DTT was also observed (Scheme 1(a) and figure 1). **P1-DTT** is in equilibrium with all other components of the exchange mixture and is constantly regenerated during the exchange process. Interestingly, at pH 8, the proportion of **P1-DTT** at equilibrium is only residual while at pH 9 it is not even detectable. However, at this later pH, and long time after equilibration, decreases of both **P1-Daa1-P2** and **P1-Daa1-P3** are observed and attributed to the competitive hydrolysis of thioester intermediates leading to the C-terminal free carboxylic peptide LYKG. Thus, in order to keep the fastest exchange rate and to avoid hydrolysis, one can consider that the optimal pH value for this dynamic process is comprised between 7 and 8. Finally, as already described in the literature for dynamic covalent libraries involving individual pH-dependent mechanisms, the inventors observed that the equilibrium constant (K) is also strongly affected by the medium, in particular when varying pH (and/or temperature), and leads to differential expressions of the products at thermodynamic equilibrium for each set of conditions.

Although leading to the first experimental example of a dynamic NCL mechanism, the N-(2-thioethyl)-cysteine incorporated as a reversible junction is not by itself a natural amino acid. The inventors have thus probed further which minimal modification of cysteine could lead to a reversible amide junction on a practical time scale while getting even closer to natural peptides. In particular, reasoning that the cis conformation could be favored even by an elementary steric hindrance on the nitrogen atom, investigations were turned to N-(methyl)-cysteine to support at least the reaction made using N-(alkyl)-cysteine. Similarly to N-(2-thioethyl)-cysteine, N-alkyl and in particular N-(methyl)-cysteine promote the rearrangement of peptide into thioesters, presumably by N→S acyl shifts under acidic conditions. For instance, this residue has been used to generate peptide thioesters in situ for native chemical ligation in the synthesis of small proteins. More recently, N-(methyl)-cysteine has also been shown to promote the N→S acyl shift in the mechanistic studies of model inteins (J. Binschik, H. D. Mootz, Angew. Chem. Int. Ed. 2013, 52, 4260-4264). Interestingly, N-(methyl)-cysteine is a nonribosomal amino acid that can be found in natural products like thiocoraline anticancer peptides. N-methylation is also a common post-translational modification found in many microbial peptides such as cyclosporins. Furthermore, the incorporation of N-methyl amino acids into peptides have been shown to improve proteolytic stability, to increase selectivity in biomolecular recognition processes, to inhibit amyloid and polyglutamine peptide aggregation, and to promote the diffusion of peptides through the blood-brain barrier. The use of N-(methyl)-cysteine, and more generally of N-(alkyl)-cysteine, to provide dynamic NCL is thus of broad interest and high potential.

N-(methyl)-cysteine **(Daa2)** was incorporated in model peptides **P1-Daa2-P2** and **Daa2-P3** which were subsequently equilibrated in solution at various pH and temperatures.

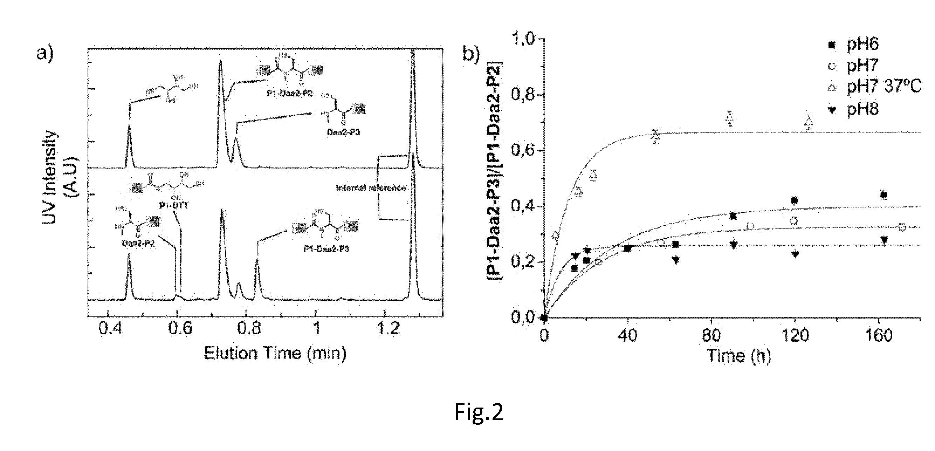

The reaction was followed by UPLC^{®}-MS (Figure 2a) and, for each time point, an aliquot of the exchange mixture was diluted with a solution containing tris(2-carboxyethyl)phosphine (TCEP) - for the characterization of the eluted peptides in their reduced thiol forms - and containing an internal UV standard (3,5-dimethoxybenzoic acid). Satisfyingly, the exchange products **P1-Daa2-P3** and **Daa2-P2** were shown to exchange at room temperature for pH 6, 7, and 8, giving initial rates (*V₀*) of 0.04, 0.05, and 0.15 mM.h⁻¹; and half-time of equilibration (*t*_{*1*/*2*}) of 25, 20, and 6.0 h respectively (Figure 2b). The average rates of the reactions in the presence of **Daa2** are thus slightly slower than in the presence of **Daa1,** which is not surprising regarding the reduced effective molarity of the internal monothiol when using N-(methyl)-cysteine instead of bisthiol N-(2-thioethyl)-cysteine. Interestingly, this decrease might also explain the only residual expression of thioester **P1-DTT** in the case of **Daa2** at pH=6, leaving the library almost fully displaced towards its condensed peptides while exchanging their fragments at all pHs. Finally, the dynamic of the system was studied at pH 7 and at 37 °C, revealing a set of kinetic parameters (*t*_{*1*/}*₂ =* 10 h and *V₀ =* 0.18 mM.h⁻¹) which ranges in a reasonable time scale to extend dynamic covalent chemistry with peptides in biologically compatible conditions.

In conclusion, modifications of a cysteine residue in polypeptides have been used to activate this specific peptide bond for exchange reactions. This dynamic design has been here refined up to the minimal incorporation of a biologically relevant N-(alkyl)-cysteine (N-(methyl)-cysteine). The time scale of the exchange reaction in physiological conditions and in the presence of DTT appears compatible with a broad range of bioassays and beyond. Importantly, this methodology allows for a high degree of control on the location of the reversible junctions in dynamic peptides or proteins or analogs thereof, i.e. molecules comprising or bearing dynamic peptides or proteins in their structure, as the process is entirely orthogonal to the other peptide bonds. Because the original NCL methodology can be further used to create peptide bonds even without a cysteine residue, as long as thiol groups are introduced by the intermediate of N-alkyl side chains (such as in **Daa1**), one can reasonably consider that the invention concept would also be applicable to other amino acids. The described concept of the dynamic NCL can be extended to "cysteine free" peptides or amides and be of great interest for the incorporation of exchangeable junctions anywhere in a protein sequence or for applications in materials science where amide bonds are often instrumental. Considering that the spontaneous rearrangement of ribosomal cysteine peptide in its thioester form does occur at a very small extent under standard conditions, this method might produce exchange reactions with any cysteine-containing peptide. Overall, this new methodology and its potential developments is considered to be of broad and great interest for the finding of therapeutically or biologically active ingredients, and/or for the study of complex chemical systems, and for the development of dynamic functional (bio)nanomaterials, typically such as dynamic polymers.

In the figures
Figure 1a) represents UPLC^{®} chromatograms of the exchange reaction between **P1-Daa1-P2** and **Daa1-P3** at pH 6 and at t=0 (*top*) and t=46 h (*bottom*).
Figure 1(b) represents the concentration ratio of peptide **P1-Daa1-P3** *vs* **P1-Daa1-P2,** as a function of time, and starting from solutions of **P1-Daa1-P2** and **Daa1-P3** at a concentration of 2.5 mM and at pH 6, 7, 8, and 9 (20°C), in a phosphate buffer (200 mM and 0.38% w/v of DTT). The trend lines are used simply to guide the reader's eye. Concentrations and error bars were calculated from calibration curves and uncertainties on experimental volumes (see examples).
Figure 2(a) represents UPLC^{®} chromatograms of the exchange reaction between **P1-Daa2-P2** and **Daa2-P3** at pH 7 and at t=0 (*top*) and t=98 h (*bottom*)*.*
Figure 2(b) represents the concentration ratio of peptide **P1-Daa2-P3** *vs* **P1-Daa2-P2,** as a function of time, and starting from solutions of **P1-Daa2-P2** and **Daa2-P3** at a concentration of 2.5 mM and at pH 6, 7, 8 (20°C), and at pH 7 (37°C), in a phosphate buffer (200 mM and 0.38% w/v of DTT). The trend lines are used simply to guide the reader's eye. Concentrations and error bars were calculated from calibration curves and uncertainties on experimental volumes (see examples).

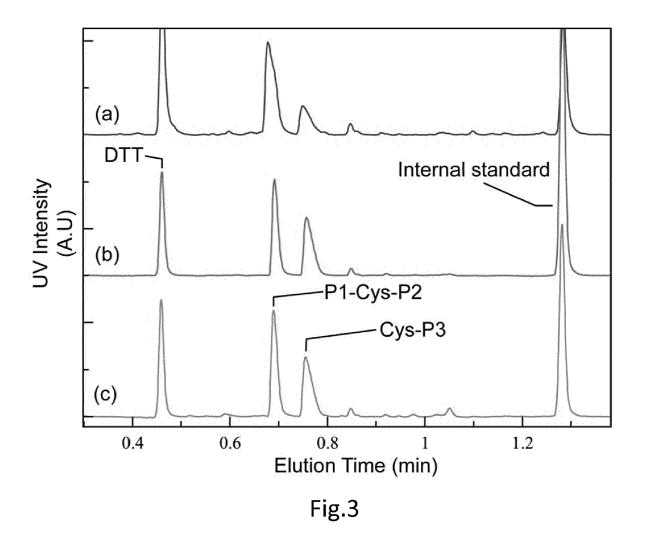

Figure 3 represents UPLC^{®} UV chromatograms of an exchange reaction between **P1-Cys-P2** and **Cys-P3** after 5min (a), 7 days (b) and 4 weeks (c) setup at pH 7 as described above, and measured from an aliquot (25µL) which was diluted with 175µL of a dilute TCEP solution containing an internal UV standard (40µL 3,5-dimethoxybenzoic acid solution (5mM in a 0.2M pH7 phosphate buffer) and 960µL of a 10mM TCEP*HCl solution in water).

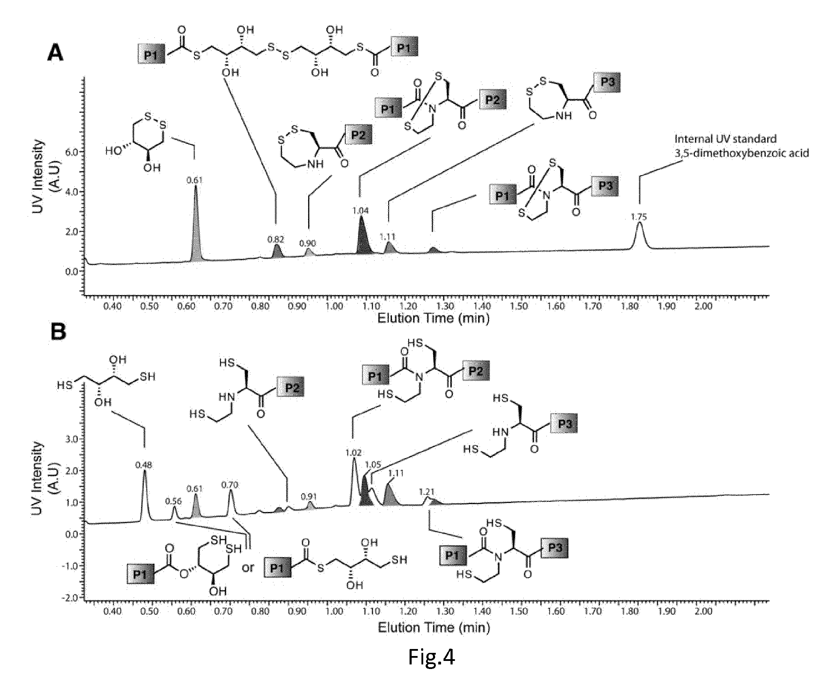

Figure 4 represents **A** UPLC^{®} UV chromatogram of an exchange reaction between **P1-Daa1-P2** and **Daa1-P3** setup as described in the experimental section and oxidation treatment of an aliquot (50mL) with H₂O₂ by dilution with 350µL of H₂O₂ containing 3,5-dimethoxybenzoic acid as an internal UV standard. **B** UPLC^{®} UV chromatogram of a sample of an exchange reaction between **P1-Daa1-P2** and **Daa1-P3** setup as described in the experimental section and diluted with 350µL of H₂O. Peaks were assigned using the corresponding mass spectrum data. The peaks at 056 and 0.70 min showed similar response by MS detection with observed molecular weights of 616.43 and 616.51 D respectively corresponding to the (M+H)⁺ ion of the two possible isomers of **P1-DTT.**

### Examples

### 1. Detailed mechanisms involving N-(2-thioethyl)-cysteine

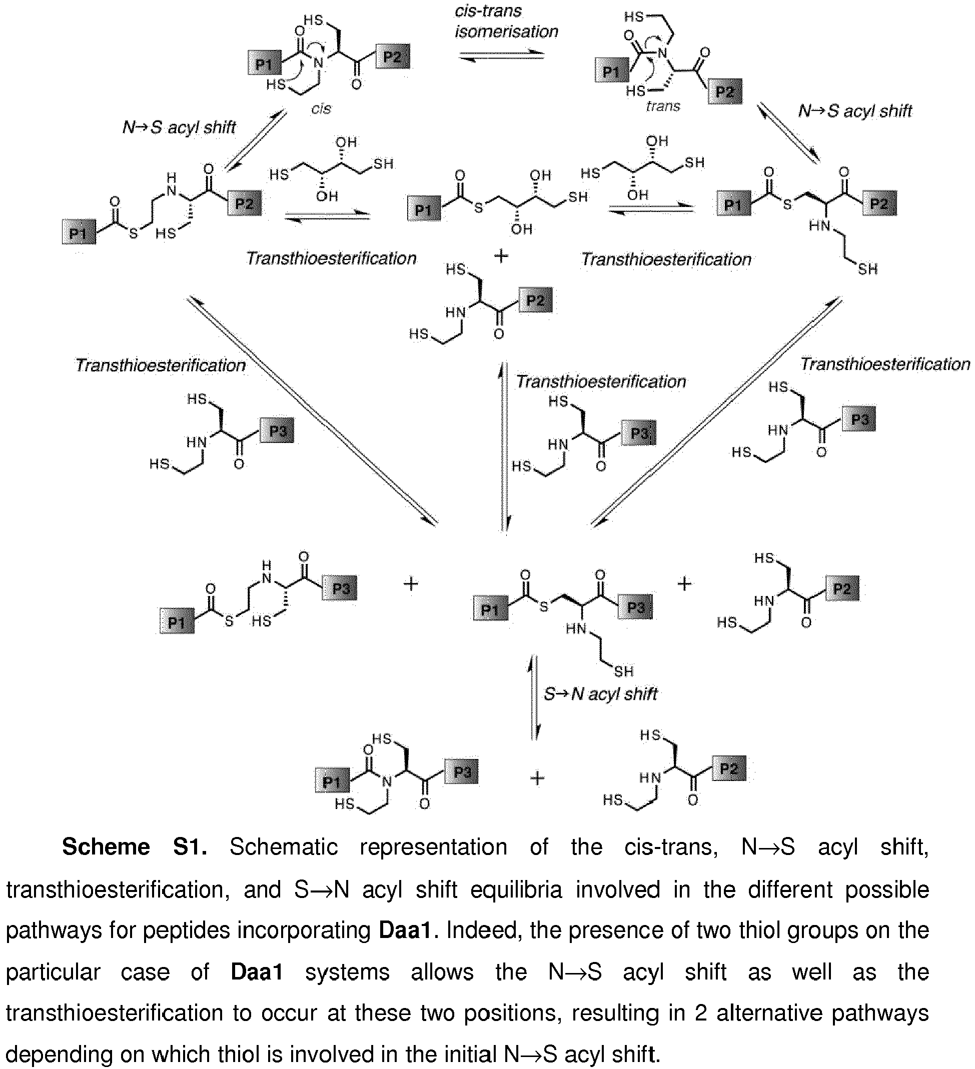

### 2. Materials and Methods

### 2.1 Generalities

**Reagents and Analytical Instruments.** All reagents and solvents were purchased from Sigma-Aldrich, Carlo Erba, Iris-Biotech, Alfa Aesar and Fisher Scientific at the highest commercial quality and used without further purification unless stated otherwise. Dry solvents were obtained using a double column *SolvTech* purification system. Peptide synthesis was performed on a microwave synthesizer (CEM Liberty 1™, Saclay, France) and on a parallel synthesizer (Heidolph^{®} Synthesis 1). Peptides were purified by High-Performance Liquid Chromatography (HPLC) Waters AutoPurification^{®} system, coupled to a Waters 2489 UV/Visible detector and Waters SQD 3100 electrospray mass detector. Purification was performed using 0.1% formic acid in water and 0.1% formic acid in methanol as the two mobile phases on either a preparative SunFire^{®} Prep C18 QBD^{®} 5µm 19x150mm column from Waters or a preparative XBridge^{®} Prep C18 5µm QBD^{®} 19x150mm column from Waters. The purity of the isolated peptides and the kinetics of the exchange reactions were monitored using a Waters ACQUITY UPLC^{®} UltraPerformance Liquid Chromatography coupled to a SQD ACQUITY Electrospray mass detector (UPLC^{®}-MS). The analysis were performed on an ACQUITY UPLC^{®} HSS T3 1.8µm 2.1x50mm column from Waters using 0.1% formic acid in water and 0.1% formic acid in acetonitrile as the two mobile phases. The processing of the UPLC^{®}-MS data (baseline subtraction and peak integration) was performed with the peak analyzer tool of OriginPro 9.0^{®}. ¹H NMR spectra were recorded (as indicated) either on a *Bruker Avance 400* spectrometer at 400 MHz or on a *Bruker Avance 500* spectrometer at 500 MHz and ¹³C NMR spectra either at 100 MHz or 125 MHz. The spectra were internally referenced to the CH₃- signals of *t*BuOH (1.24ppm for ¹H NMR and 30.29ppm for ¹³C NMR) (H. E. Gottlieb, V. Kotlyar, A. Nudelman, J. Org. Chem. 1997, 62, 7512-7515), the chemical shifts are given in ppm. Spin multiplicities are reported as a singlet (s), doublet (d), doublet of doublets (dd), triplet (t) and quartet (q) with coupling constants (J) given in Hz, or multiplet (m). Broad peaks are marked as br. When specified, ¹H resonances were assigned with the aid of additional information from 2D NMR proton correlation spectroscopy spectra (H-COSY). ESI-MS spectra were acquired using the *Waters SQD* Mass Detector directly connected to the output of an UPLC^{®} or HPLC column.

**Abbreviations.** aa: amino acid; *DBU:* 1,8-Diazabicyclo[5.4.0]undec-7-ene; DCM: Dichloromethane; DIAD: Diisopropyl azodicarboxylate; DIEA: N,N-Diisopropylethylamine; DIPCDI: N,N'-Diisopropylcarbodiimide; *DMF: N,N-Dimethylformamide; DTT:* Dithiothreitol; Fmoc: 9-Fluorenylmethoxycarbonyl; HATU: 1-[Bis(dimethylamino)methylene]-1H-1,2,3-triazolo[4,5-b]pyridinium 3-oxid hexafluorophosphate; *HBTU:* N,N,N',N'-Tetramethyl-O-(1H-benzotriazol-1-yl)uronium hexafluorophosphate; MBHA: methylbenzhydrylamine; NMP: N-Methyl-2*-pyrrolidone; Oxyma Pure*^{®}*:* Ethyl 2-cyano-2-(hydroxyimino)acetate; rt: room temperature; TBAF: Tetra-n-butylammonium fluoride; TCEP: *tris(2-*carboxyethyl)phosphine; THF: Tetrahydrofuran; TIS: Triisopropylsilane; TFA: Trifluoroacetic acid.

### 2.2 Syntheses

Standard solid phase peptide synthesis was performed using standard Fmoc/*tert-*butyl chemistry (W. Chan, P. White, Fmoc Solid Phase Peptide Synthesis: A Practical Approach, Oxford University Press, USA, 2000) and using Microwave assisted (S. L. Pedersen, A. P. Tofteng, L. Malik, K. J. Jensen, Chem. Soc. Rev. 2012, 41, 1826-1844) solid phase peptide synthesis with a CEM Liberty 1™ microwave synthesizer starting with a Fmoc-Rink-amide MBHA resin (0.74 mmol/g). A typical synthesis was done on a 0.5mmol scale. For each amino acid, double couplings were performed at 70ºC and 35W (microwave power) for 5min (except for the coupling of Fmoc-Cys(Trt)-OH which is performed without microwave heating for 2min and then at 50ºC with 25W for 4min) using 4-fold molar excess of each Fmoc L-aa (10mL of a 0.2M solution in DMF), HBTU (4mL of a 0.5M solution in DMF) and DIEA (2mL of a 2M solution in NMP). Fmoc groups were deprotected with 2 successive treatments with 20vol% piperidine solution in DMF (15mL, 70ºC for 3min, 55W).

### 2.2.1 Peptides incorporating the N-(2-thioethyl)-cysteine

A representative synthesis for this family of peptides is given in Scheme S2. The C-terminal peptide portion (-CAKLL-NH₂ **P2** or -CAFKF-NH₂ **P3**) of all peptides was synthesized using the standard Fmoc-solid phase peptide synthesis described above. The strategy used for the introduction of the N-(2-thioethyl) group on the cysteine was adapted from a protocol described by Hou *et al.* for the modification of primary amines on solid support (W. Hou, X. Zhang, F. Li, C.-F. Liu, Org. Lett. 2011, 13, 386-389). After the standard deprotection of the Fmoc group at the terminal cysteine, the resin was transferred in a parallel synthesizer and the terminal amino group converted into a sulfonamide with a 2-nitrobenzenesulfonylchloride (4 eq) and 2,6-lutidine (6 eq) solution in DCM (10mL) for 3h. A Mitsunobu reaction was subsequently performed with 2-(tritylthio)ethanol (W.-S. Yeo, D.-H. Min, R. W. Hsieh, G. L. Greene, M. Mrksich, Angew. Chem. Int. Ed. 2005, 44, 5480-5483) (5 eq), triphenylphosphine (5 eq), DIAD (5 eq) in a dry THF/dry DCM mixture (5mL and 6.5mL respectively) under argon atmosphere for 5h; this reaction was then repeated once more for 12 hours. The amino group was then deprotected with 2,2'-(ethyldioxy)diethanethiol (20 eq) and DBU (10 eq) in 5mL of DMF (2 x 2h).

The following coupling, of Fmoc-Gly-OH, was performed using the Fmoc-aa (8 eq), DIPCDI (8 eq) and OxymaPure (8 eq) in DMF (5mL) at rt under argon atmosphere for 72h.

After each of these steps, a small amount of resin was submitted to a cleavage solution of TFA/TIS/2-2'(ethyldioxy)diethanethiol/H₂O: 92.5/2.5/2.5/2.5 vol% and the crude peptide isolated after precipitation in diethyl ether in order to check that the reaction went to completion using UPLC^{®}-MS analysis.

For peptides **P1-Daa1-P2** and **P1-Daa1-P3** the remaining amino acids of the N-terminal peptide portion (LYK) were then coupled using the standard Fmoc-solid phase peptide synthesis described above.

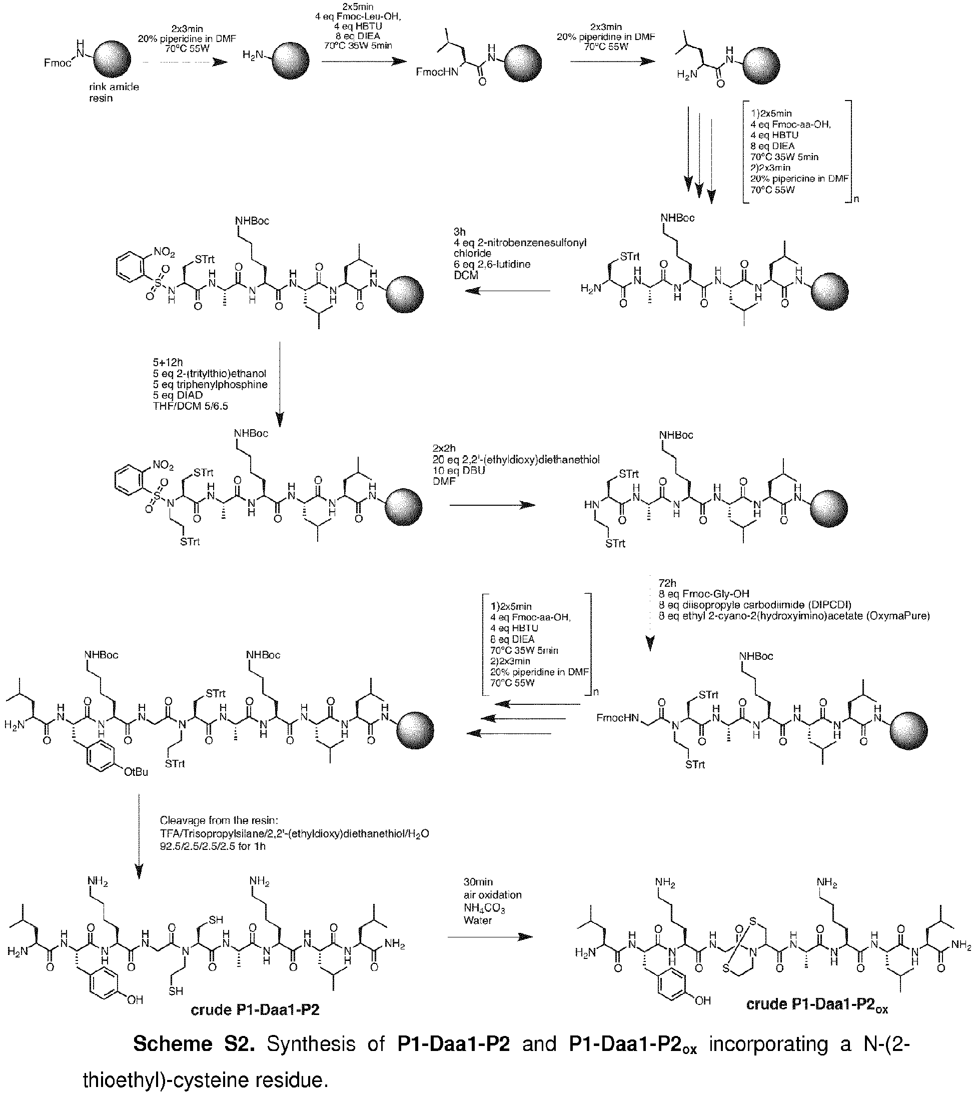

### 2.2.2 Peptides incorporating N-(methyl)-cysteine

A representative synthesis for this family of peptides is given in Scheme S3. The C-terminal peptide portion (-CAKLL-NH₂ **P2** or -CAFKF-NH₂ **P3**) of all peptides was synthesized using the standard Fmoc-solid phase peptide synthesis described above. The strategy used for the introduction of the N-(methyl) group on the cysteine was adapted from a protocol described by Erlich *et al.* for the modification of cysteine on solid support (L. A. Erlich, K. S. A. Kumar, M. Haj-Yahya, P. E. Dawson, A. Brik, Org. Biomol. Chem. 2010, 8, 2392-2396). Once the coupling of the Fmoc-Cys(Trt)-OH was achieved and the Fmoc group removed, the resin was transferred in a parallel synthesizer for shacking. The terminal amino group was activated with a 2-nitrobenzenesulfonylchloride (4 eq) and 2,6-lutidine (6 eq) solution in DCM (10mL) for 3h under argon. A methylation with methyl iodide (20 eq) and TBAF (1M solution in THF, 2.5mL) was then performed twice under argon (2 x 1 h). The amino group was then deprotected with 2-2'(ethyldioxy)diethanethiol (20 eq) and DBU (10 eq) in 5mL of DMF (2 x 2h).

The following coupling was performed in the CEM Liberty 1^{®} microwave synthesizer using Fmoc-Gly-OH (4 eq), HATU (4 eq), and DIEA (5.4 eq) in DMF (8mL) at 75° C and 30W (2 x 20min).

After each of these steps, as small amount of resin was submitted to TFA/TIS/2-2'(ethyldioxy)diethanethiol/H₂O: 92.5/2.5/2.5/2.5 vol% and the crude peptide isolated after precipitation in diethyl ether in order to check that the reaction went to completion by UPLC^{®}-MS analysis.

For peptides **P1-Daa2-P2** and **P1-Daa2-P3** the remaining amino acids of the N-terminal peptide portion (LYK) were then coupled using the standard Fmoc-solid phase peptide synthesis described above.

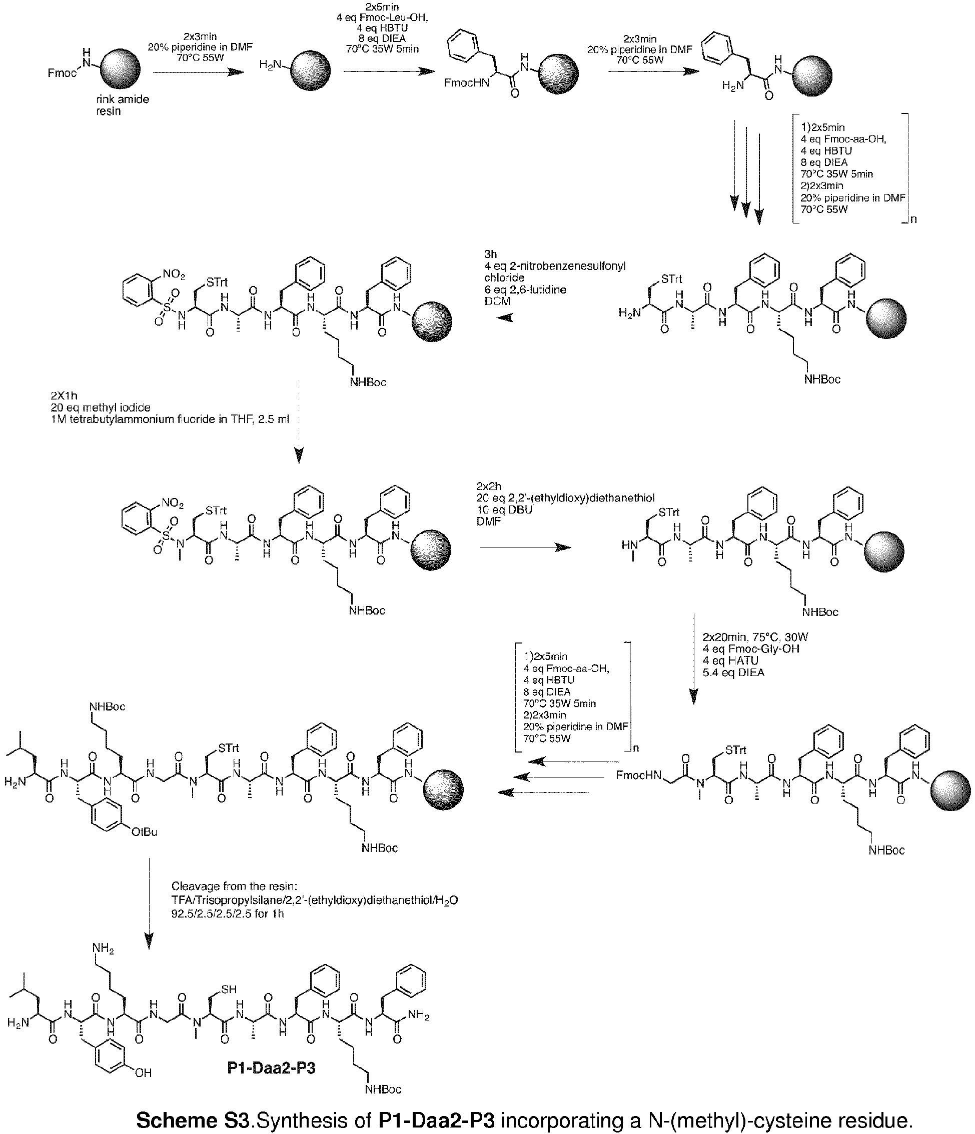

### 2.2.3 Unmodified cysteine peptides for control experiments

These peptides were synthesized according to standard protocols for the Fmoc-solid phase peptide synthesis described above.

### 2.3 Final cleavages of the peptides from the resin and purification

The resin was suspended in a 10mL solution of TFA/TIS/2-2'(ethyldioxy)diethanethiol/H₂O: 92.5/2.5/2.5/2.5 vol%. After 1 h, the resin was filtered and washed with TFA (5mL). The combined filtrates were concentrated *in vacuo* and then added to cold diethylether (200mL) to precipitate the crude peptides. The solids were separated by centrifugation, washed with cold diethylether (2 x 40mL) and dried under argon to afford crude peptides.

Crude peptides were then purified by High-Performance Liquid Chromatography (HPLC) Waters AutoPurification^{®} system, coupled to a Waters 2489 UV/Visible detector and Waters SQD 3100 electrospray mass detector. Purification was performed using 0.1% formic acid in water (A) and 0.1% formic acid in methanol (B) as the two mobile phases on either a preparative SunFire^{®} Prep C18 QBD™ 5µm 19x150mm column from Waters or a preparative XBridge^{®} Prep C18 5µm QBD™ 19x150mm column from Waters.

A typical gradient is given here:

| Time (min) | Flow (ml/min) | %A | %B |
|---|---|---|---|
| 0 | 24.5 | 95 | 5 |
| 0.95 | 24.5 | 95 | 5 |
| 25 | 24.5 | 5 | 95 |
| 33 | 24.5 | 5 | 95 |
| 34 | 24.5 | 95 | 5 |

For all peptides, fractions were automatically collected using the ESI-MS signal of the peptide as a trigger. Fractions were individually analyzed using Waters ACQUITY UPLC^{®} Ultra Performance Liquid Chromatography coupled to a SQD ACQUITY Electrospray mass detector (UPLC^{®}-MS). The analysis were performed on an ACQUITY UPLC^{®} HSS T3 1.8µm 2.1x50mm column from Waters using 0.1% formic acid in water and 0.1% formic acid in acetonitrile as the two mobile phases. The organic solvent (methanol or acetonitrile) was removed under reduced pressure from the combined fractions before lyophilization of the solution of the purified peptides.

### 2.3.1 Oxydation of N-(2-thioethyl)-cysteine

For the peptides incorporating N-(2-thioethyl)-cysteine, an additional oxidation step was performed on the crude products before injection in preparative HPLC. They were dissolved in milliQ H₂O (1mL/50mg), the pH was adjusted to 7-8 with (NH₄)₂CO₃ and compressed air was bubbled in the resulting solution for 30 min. Prior to purification, the pH was lowered to 4-5 with formic acid. This procedure was used to avoid complications afforded by the purification of these peptides in their corresponding reduced form, which can undergo oxidation during the purification process.

### 2.4 Protocols for exchange reactions

### 2.4.1 Control Exchange on Cysteine Native Peptides

A control exchange under identical conditions at pH 7 between the native peptides **P1-Cys-P2** and **Cys-P3** did not yield any detectable trace of exchange products **P1-Cys-P3** and **Cys-P2** after 4 weeks (Figure 3).

The 0.2M phosphate buffer was prepared at pH 7: the amount of NaH₂PO₄ required to give a 200mM solution in 20mL of water (551 mg) was dissolved in 10mL of water. The resulting acidic solution was titrated to pH 7 using 2M NaOH and its volume adjusted to 20mL to give the 0.2M buffer solution at this pH. The buffer was degassed using freeze/thaw cycles and placed under argon prior to use.

**Cys-P3** (formate salt) was dissolved in water to give a solution **A** (5mM). **P1-Cys-P2** (diformate salt) was dissolved in water to give a solution **B** (5mM). 250µL of solution **A** were mixed with 250µL of solution **B** and were immediately frozen and lyophilized. The lyophilized powder of the mixed peptides was diluted under argon with 500µL of phosphate buffer (pH 7) containing 25mM of DTT (5.8mg in 1.5mL).

### 2.4.2 N-(2-thioethyl)-cysteine modified peptides

0.2M phosphate buffers were prepared at pH 6, 7, 8 and 9: the amount of NaH₂PO₄ required to give a 200mM solution in 20mL of water (551 mg) was dissolved in 10mL of water. The resulting acidic solution was titrated to pH 6, 7, 8, or 9 using 2M NaOH and its volume adjusted to 20mL to give 0.2M buffer solutions at these pHs. Buffers were degassed using freeze/thaw cycles and placed under argon prior to use.

**Daa1-P3** (formate salt) was dissolved in water to give a solution **A** (5mM). **P1-Daa1-P2** (diformate salt) was dissolved in water to give a solution **B** (5mM). 250µL of solution **A** were mixed with 250 µL of solution **B** and diluted with a solution C of TCEP (10mM, 7.1 mg in 2.5mL of water neutralized with 8.4mg of NaHCO₃, 2 eq., 250mL) to give a solution D. This solution **D** was immediately frozen and lyophilized. The lyophilized powder of the mixed peptides was diluted under argon with 500µl of phosphate buffer (pH 6, 7, 8 or 9) containing 25mM of DTT (5.8mg in 1.5 mL)

A control exchange was setup at pH 7 as described above but starting from **P1-Daa1-P3** and **Daa1-P2.** As expected this reaction led to the unambiguous formation of **P1-Daa1-P2** and **Daa1-P3** resulting from a dynamic native chemical ligation process at the Daa1 amino acid.

When aliquots of the reaction were diluted with water prior to UPLC^{®}-MS analysis, the overlapping of the different peaks corresponding to the oxidized and reduced forms of the 4 peptides in equilibrium prevented a quantitative analysis of the reaction kinetics (Figure 4)

In order to monitor these exchange reactions, for each time point an aliquot of the reaction (25µL) was diluted with 175µL of a dilute H₂O₂ solution containing an internal UV standard (23µL H₂O₂ (30% solution), 20µL 3,5-dimethoxybenzoic acid solution (5mM in a 0.2M pH7 phosphate buffer) and 957µL water). The resulting solution was filtered and injected in the UPLC^{®}-MS (2 x 2µL injections).

### 2.4.3 N-(methyl)-cysteine modified peptides

0.2M phosphate buffers were prepared as described above at pH 6, 7 and 8. They were degassed and stored under Ar.

**Daa2-P3** (formate salt) was dissolved in water to give a solution **A** (5mM). **P1-Daa2-P2** (diformate salt) was dissolved in water to give a solution **B** (5mM). 250µL of solution **A** were mixed with 250µL of solution **B** and were immediately frozen and lyophilized. The lyophilized powder of the mixed peptides was diluted under argon with 500µL of phosphate buffer (pH 6, 7 or 8) containing 25mM of DTT (5.8mg in 1.5mL).

In order to monitor these exchange reactions, for each time point an aliquot of the reaction (25µL) was diluted with 175 µl of a dilute TCEP solution containing an internal UV standard (40µL 3,5-dimethoxybenzoic acid solution (5mM in a 0.2M pH7 phosphate buffer) and 960µL of a 10mM TCEP*HCl solution in water). The resulting solution was filtered and injected in the UPLC^{®}-MS (2 x 2µL injections).

### 2.5 Calibration curves and error determinations

Calibration curves were established for peptides **P1-Daa1-P2, P1-Daa1-P3, P1-Daa2-P2,** and **P1-Daa2-P3** by injecting 2µL of the peptides at known concentrations (62.50, 125.00, 156.25, 312.50, 500.00 and 625.00µM) under conditions identical as the ones used for the dilution of the aliquots of the exchange reactions. After a manual baseline correction with the peak analyzer tool of OriginPro 9.0^{®} the area of the peak corresponding to the peptide was then plotted against the concentration. Each standard point corresponds to an average of 3 replicates. A linear regression analysis then gave 4 linear calibration curves with R² between 0.99324 and 0.99924 that where then used to calculate the concentration of the peptides in the exchange mixtures.

### 3. Peptides Characterizations

The purity of the isolated peptides and the kinetics of the exchange reactions were monitored using a Waters ACQUITY UPLC^{®} UltraPerformance Liquid Chromatography coupled to a SQD ACQUITY Electrospray mass detector (UPLC-MS). The analysis were performed on an ACQUITY UPLC^{®}HSS T3 1.8µm 2.1x50mm column from Waters using 0.1% formic acid in water (eluent A) and 0.1% formic acid in acetonitrile (eluent B) as the two mobile phases and the following gradient:

| Time (min) | Flow (ml/min) | %A | %B |
|---|---|---|---|
| 0 | 1 | 95 | 5 |
| 3.5 | 1 | 5 | 95 |
| 4 | 1 | 5 | 95 |
| 4.1 | 1 | 95 | 5 |
| 5.1 | 1 | 95 | 5 |

The processing of the UPLC^{®} UV data (baseline subtraction and peak integration) was performed with the peak analyzer tool of OriginPro 9.0^{®}.

ESI-MS spectra were acquired using the Waters SQD Mass Detector directly connected to the output of an UPLC^{®} or HPLC column.

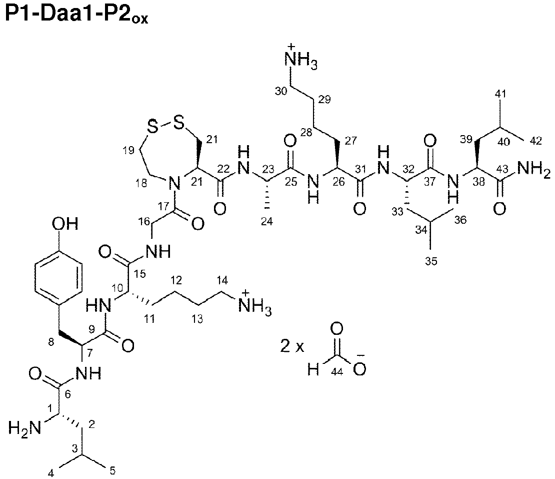

¹H NMR (500 MHz D₂O): 8.46 (bs, 2H, 2 H-C(44)), 7.14 (d, 2H, aromatic protons Tyr), 6.83 (d, 2H, aromatic protons Tyr residue), 4.59 (dd, 1 H, J= 9.7 Hz, 6.8 Hz), 4.40-4.15 (m, 7H), 4.05-3.93 (m, 2H), 3.88-3.76 (m, 1 H), 3.55-3.32 (m, 2H), 3.21-2.87 (m, 8H), 1.85-1.53 (m, 17H), 1.50-1.28 (m, 7H), 0.99-0.84 (m, 18H).

¹³C NMR (125 MHz, D₂O): 177.98, 175.94, 175.13, 174.41, 173.54, 172.89, 172.21, 171.65, 170.76, 163.90, 163.62, 155.31, 131.31, 128.20, 118.21, 116.24, 115.89, 56.19, 54.17, 53.72, 53.57, 53.07, 52.75, 52.34, 50.89, 40.58, 40.42, 40.16, 39.88, 39.82, 36.81, 31.33, 30.91, 26.96, 26.92, 25.04, 25.01, 24.51, 22.95, 22.80, 22.55, 22.29, 22.00, 21.48, 21.21, 17.16.

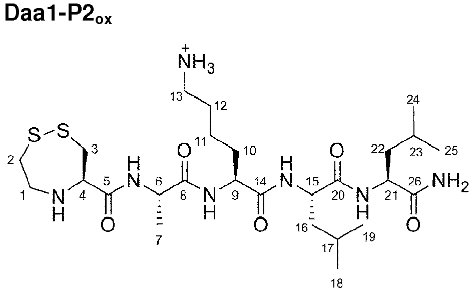

¹H NMR (500 MHz D₂O): 4.41-4.22 (m, 4H, H-C(6) + H-C(9) + H-C(15) + H-C(21)), 4.11-4.00 (m, 1H, H-C(4)), 3.62-3.45 (m, 2H, 1 H-C(1) + 1 H-C(3)), 3.23-3.09 (m, 2H), 3.07-2.89 (m, 4H), 1.86-1.51 (m, 10H, 2 H-C(11) + 2 H-C(12) + 2 H-C(16) + 1 H-C(17) + 2 H-C(22) + 1 H-C(23)), 1.51-1.40 (m, 2H, 2 H-C(10)), 1.40-1.31 (d, 3H, 1 H-C(7) J=7.50), 1.08-0.69 (m, 12H, 3 H-C(18) + 3 H-C(19) + 3 H-C(24) + 3 H-C(25)).

¹³C NMR (125 MHz, D₂O): 178.00, 175.64, 175.13, 174.31, 63.07, 54.12, 53.01, 52.76, 50.53, 49.54, 40.42, 40.14, 39.88, 30.97, 26.97, 25.04, 25.02, 22.93, 22.81, 22.76, 21.45, 21.24, 17.17 (C7).

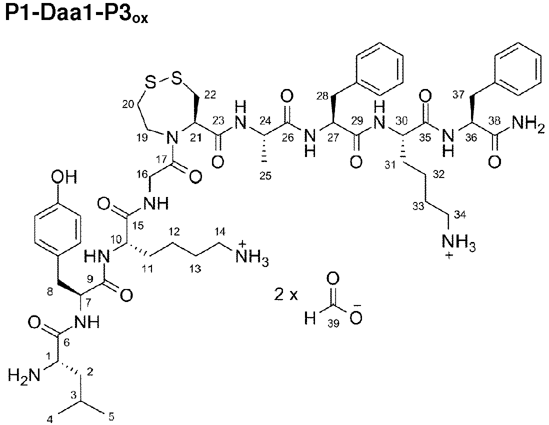

¹H NMR (500 MHz D₂O): 8.46 (s, 1H, 1 H-C(39)), 7.41-7.18 (m, 10H, aromatic protons Phe), 7.13 (d, 2H, 2 aromatic protons Tyr J=8.6Hz), 6.83 (d, 2H, 2 aromatic protons Tyr, J=8.6Hz), 4.67-4.50 (m, 4H), 4.37-4.16 (m, 5H), 4.04-3.95 (m, 2H), 3.81-3.69 (m, 1 H), 3.43-3.27 (m, 2H), 3.23-2.87 (m, 13H), 1.82-1.50 (m, 12H), 1.41-1.27 (m, 4H), 1.22-1.11 (m, 2H), 0.99-0.88 (m, 3 H-C(4) + 3 H-C(5)).

¹³C NMR (100 MHz, D₂O): 176.20, 175.57, 173.64, 173.61, 173.52, 172.90, 172.10, 171.48, 163.91, 163.63, 155.30, 137.14, 136.70, 131.31, 129.96, 129.93, 129.54, 129.48, 128.22, 128.04, 127.89, 118.20, 116.23, 115.89, 56.14, 55.53, 55.34, 54.16, 53.58, 52.39, 50.83, 41.45, 40.72, 39.82, 39.79, 38.88, 37.59, 37.28, 36.78, 31.31, 31.04, 26.97, 26.91, 24.52, 22.55, 22.53, 22.30, 22.02, 16.59.

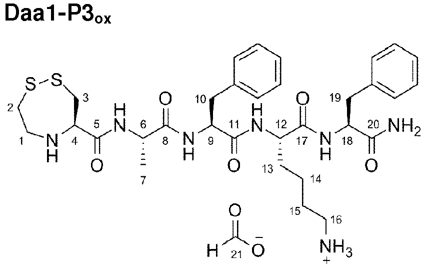

¹H NMR (400 MHz D₂O): 8.45 (s, 1H, H-C(21)), 7.42-7.15 (m, 10H, aromatic protons), 4.59-4.50 (m, 2H, H-C(9) + H-C(18)), 4.27-4.16 (m, 2H, H-C(6) + H-C(12)), 3.86 (dd, 1 H, H-C(4) J=10.75 Hz, 4.27 Hz), 3.52-3.42 (m, 1 H, 1 H-C(1)), 3.34 (dd, 1 H, 1 H-C(3) J=13.48 Hz, 4.27 Hz), 3.22-2.88 (m, 9H, 1 H-C(1) + 2 H-C(2) +2 H-C(10) + 2 H-C(16) + 2 H-C(19)), 2.81 (dd, 1 H, 1 H-C(3) J=13.48 Hz, 10.75 Hz), 1.68-1.50 (m, 4H, 2 H-C(13) +2 H-C(15)), 1.31 (d, 3H, 3 H-C(7) J=7.17 Hz), 1.26-1.14 (m, 2H, 2H-C(14)).

¹³C NMR (125 MHz, D₂O): 176.19, 174.88, 173.39, 173.30, 171.54, 137.07, 136.61, 129.96, 129.88, 129.50, 129.48, 127.94, 62.84, 55.51, 55.43, 53.88, 50.42, 49.37, 39.82, 37.67, 37.64, 31.21, 26.94, 22.48, 17.12 (C-7).

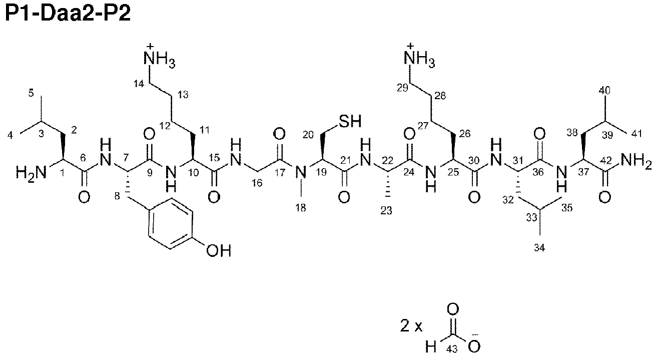

¹H NMR (500 MHz, D₂O): 8.29 (s, excess formic acid to slow down oxidation), 7.15 (d, 2H, aromatic protons, J=8.1 Hz), 6.81 (d, aromatic protons), 5.04 (dd, 1 H, J=8.3Hz, 6.4Hz), 4.63-4.57 (m, 1 H), 4.40-4.22 (m, 5H), 4.08-3.97 (m, 2H), 3.27-2.11 (m, 12H), 1.89-1.53 (m, 18H), 1.53-1.30 (m, 6H) 1.02-0.81 (m, 18H).

¹³C NMR (125 MHz, D₂O): 177.99, 175.72, 175.14, 174.37, 173.68, 172.97, 171.63, 171.48, 170.73, 155.33, 131.33, 128.13, 116.22, 60.72, 56.22, 54.25, 53.65, 53.04, 52.77, 52.34, 50.90, 40.57, 40.41, 40.16, 39.87, 39.81, 31.54, 31.29, 30.95, 26.98, 26.92, 25.03, 25.01, 24.52, 22.94, 22.79, 22.54, 22.27, 22.02, 21.43, 21.20, 17.06.

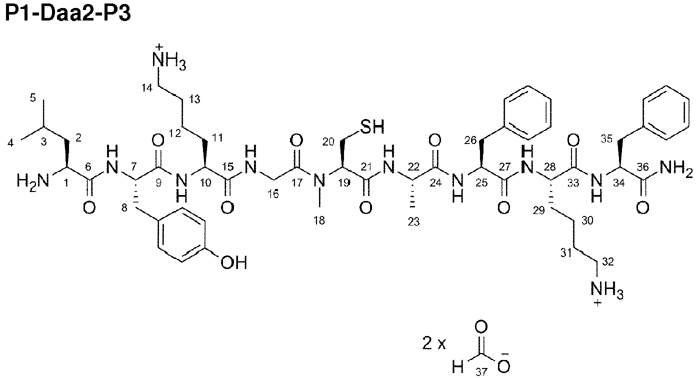

¹H NMR (500 MHz D₂O): 7.41-7.22 (m, 8H, aromatic protons Phe), 7.21-7.08 (m, 4H, aromatic protons Phe+Tyr), 6.86-6.75 (m, 2H, aromatic protons Tyr), 4.96 (dd, 1 H, J=9Hz, 6.3Hz), 4.59 (dd, 1 H, J=9.9 Hz, 6.6Hz), 4.57-4.48 (m, 2H), 4.39-4.14 (m, 3H), 4.11-3.94 (m, 2H), 3.23-2.68 (m, 16H), 1.85-1.47 (m, 10H), 1.46-1.11 (m, 8H), 1.03-0.86 (m, 6H, 3 H-C(4) + 3 H-C(5)).

¹³C NMR (125 MHz, D₂O): 176.21, 175.32, 173.67, 173.52, 173.43, 172.96, 171.64, 171.43, 170.72, 155.32, 137.11, 136.64, 131.33, 129.95, 129.91, 129.50, 129.48, 128.15, 127.94, 127.91, 116.22, 60.96, 56.21, 55.53, 55.36, 54.01, 53.64, 52.34, 50.75, 42.14, 40.57, 39.83, 39.80, 37.60, 37.49, 31.77, 31.29, 31.11, 26.94, 26.92, 24.52, 22.94, 22.54, 22.52, 22.27, 22.02, 16.84.

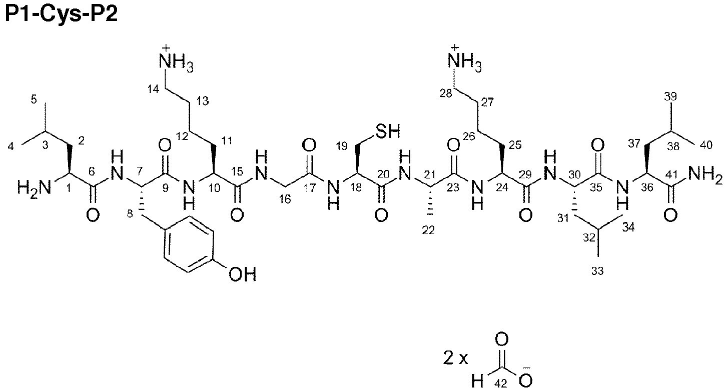

¹H NMR (500 MHz, D₂O): 7.15 (d, 2H aromatic protons Tyr, J=8 Hz), 6.83 (d, 2H, aromatic protons Tyr), 4.62 (dd, 1 H, H-C(7) Tyr J=8.9 Hz, 7.2Hz), 4.52 (t, 1 H, J=6.5Hz), 4.38-4.20 (m, 5H), 4.00 (t, 1 H), 3.89 (s, 2H, 2 H-C(16)), 3.10-2.86 (m, 8H), 1.86-1.52 (m, 18H), 1.50-1.27 (m, 7H), 0.97-0.82 (m 18H, 3 H-C(4) + 3 H-C(5) + 3 H-C(39) + 3 H-C(40)).

¹³C NMR (125 MHz, D₂O): 177.20, 174.73, 174.31, 173.50, 173.33, 172.34, 171.57, 170.73, 169.98, 154.54, 130.55, 127.42, 115.43, 55.29, 55.23, 53.46, 53.23, 52.23, 51.98, 51.53, 49.90, 42.19, 39.79, 39.62, 39.35, 39.07, 39.01, 35, 95, 30.23, 26.20, 26.18, 25.33, 24.24, 24.23, 23.71, 22.14, 22.01, 21.98, 21.78, 21.55, 21.12, 20.66, 20.44, 16.30.

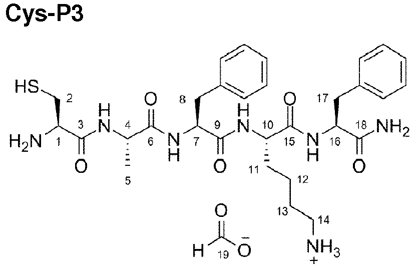

¹H NMR (400 MHz D₂O): 8.29 (s, excess formic acid to slow down oxidation), 7.43-7.10 (m, 10H, aromatic protons Phe), 4.56-4.45 (m, 2H, H-C(7) + H-C(16)), 4.44-4.31 (m, 1H), 4.25-4.13 (m, 1H), 3.36-3.27 (m, 0.5H), 3.19-2.85 (m, 7.5H), 1.68-1.49 (m, 4H), 1.37-1.31 (m, 3H, -CH3 Ala), 1.28-1.11 (m, 2H).

¹³C NMR (125 MHz, D₂O): 173.20, 173.07, 168.39, 168.24, 137.07, 137.05, 136.61, 136.59, 129.95, 129.90, 129.86, 129.51, 129.49, 129.47, 127.95, 55.70, 55.60, 55.46, 55.44, 54.70, 53.84, 53.82, 52.17, 50.37, 50.26, 39.83, 39.80, 37.99, 37.93, 37.80, 37.71, 37.65, 31.37, 31.26, 26.97, 26.94, 25.76, 22.46, 22.43, 17.55, 17.16.

To slow down oxidation of this peptide during the NMR measurement the inventors degassed the D₂O +tBuOH mixture and added an excess of formic acid to the sample. This didn't prevent oxidation completely and this compound is observed as a mixture of the reduced cysteine and disulfide peptides.

## Claims

1. A mixture of molecules comprising at least molecules (p1p2) as defined by formula (I) and at least molecules (p3) as defined by formula (II) 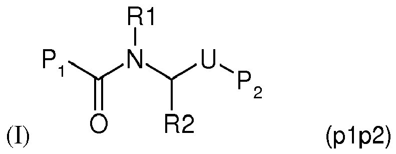 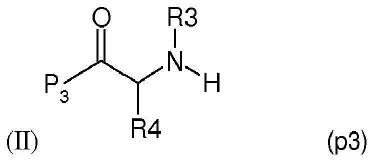
wherein R1, R2, R3, R4, P1, P2, and P3 are each independently atomic groups,
wherein at least one of R1 and R2 is -CH2(CH2)ₓ-SH wherein x is 0, or 1 to 6,
wherein at least one of R3 and R4 is -CH2(CH2)ₓ-SH wherein x is 0, or 1 to 6
wherein -U- is an aliphatic group optionally substituted, or an aromatic group optionally substituted, or -C(O)-, -O-, -S-, or a phosphorus containing function.

2. The mixture of molecules according to claim 1, wherein said mixture further comprises at least molecules (p1p3) as defined by formula (III) and at least molecules (p2) as defined by formula (IV) 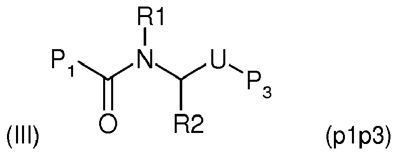 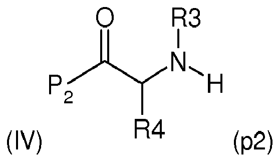 wherein R1, R2, R3, R4, P1, P2, P3 and U are as defined in claim 1.

3. The mixture of molecules according to claim 1 or 2, wherein R1 and R2 are-CH2(CH2)ₓ-SH, and/or wherein R3 and R4 are -CH2(CH2)ₓ-SH.

4. The mixture of molecules according to any one of claims 1 to 3, wherein molecules (p1 p3) and (p3) are of formula (Ia) and (IIa) as follows:

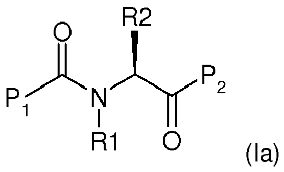

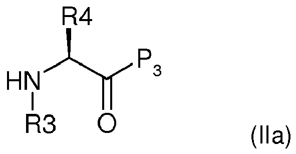

5. The mixture of molecules according to any one of claims 1 to 4, wherein R1 is H, aliphatic, including alkyl, preferably C1-6-alkyl, for example C1, C2, C3, C4, C5 or C6-alkyl, more preferably is H or methyl.

6. The mixture of molecules according to any one of claims 1 to 5, wherein R3 is H, aliphatic, including alkyl, preferably C1-6-alkyl, for example C1, C2, C3, C4, C5 or C6-alkyl, more preferably is H or methyl.

7. The mixture of molecules according to any one of claims 1 to 5, wherein P1, P2, and P3 are each independently selected from the group consisting of single unit molecules (monomers), for example therapeutic molecules, peptides, nucleotides, and polymers molecules, for example oligopeptides, polypeptides, oligonucleotides, polynucleotides, polyamides, including non-peptide polyamides, including aliphatic polyamides and aromatic polyamides.

8. The mixture of molecules according to any one of claims 1 to 5, wherein said mixture comprises more than "n" different molecules (I1) to (In) of formula (I) wherein n represent a number above 5, preferably above 10, more preferably above 100, for example above 1000, or above 10³, or above 10⁴ or above 10⁵, wherein P1 and/or P2 represent different atomic groups when comparing each different molecules (I1) to (In), thereby defining a specific structure for each of molecules (I1) to (In).

9. The mixture of molecules according to any one of claims 1 to 5, wherein said mixture comprises more than "n" different molecules (II1) to (IIn) of formula (II) wherein n represent a number above 5, preferably above 10, more preferably above 100, for example above 1000, or above 10³, or above 10⁴ or above 10⁵, wherein P3 represents different atomic groups when comparing each different molecules (II1) to (IIn), thereby defining a specific structure for each of molecules (II1) to (IIn).

10. A combinatorial library of molecules comprising a mixture as defined according to any one of claims 1 to 9.

11. Method for preparing a compound P1 P2 as defined according to any one of claims 1 to 9, said method comprising the following chemical reaction: 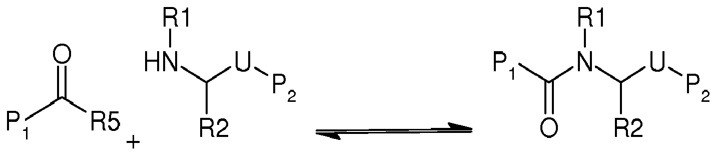
wherein R1, R2, P1, and P2 are each independently atomic groups,
wherein at least one of R1 and R2 is -CH2(CH2)ₓ-SH wherein x is 0, or 1 to 6,
wherein -U- is an aliphatic group optionally substituted, or an aromatic group optionally substituted, or -C(O)-, -O-, -S-, or ;
wherein R5 is a thioester -SR6 with R6 is a hydrogen atom or an aliphatic or aromatic group, or an amine having the chemical structure below to form an amide together with -C(O)P1: 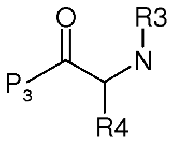
wherein R3, and R4 are each independently atomic groups,
wherein at least one of R3 and R4 is -CH2(CH2)ₓ-SH wherein x is 0, or 1 to 6.

12. Method for preparing a mixture as defined according to any one of claims 1 to 9, said method comprising the following chemical reaction: 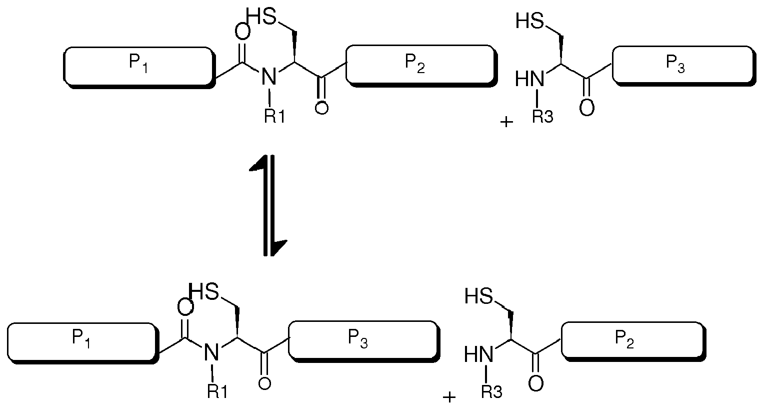 wherein R1, R2, R3, R4, P1, P2, and P3 are as defined in any one of claims 1 to 9.

13. Method for preparing a mixture as defined according to any one of claims 1 to 9, said method comprising the following chemical reaction: 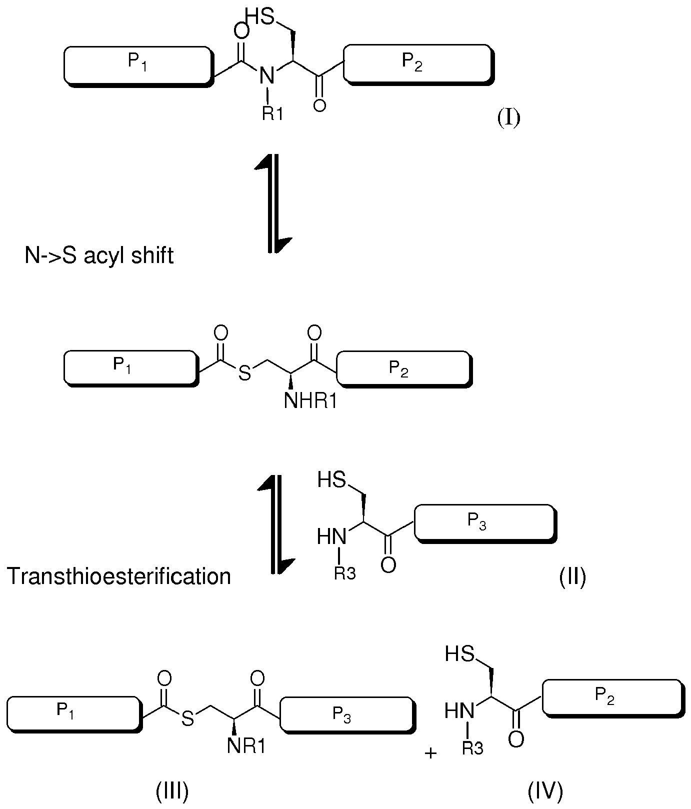 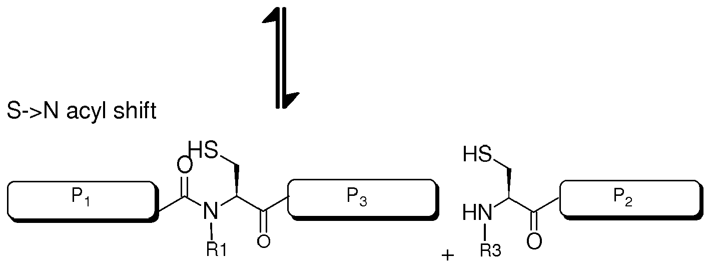
wherein P1, P2, P3 are polymers,
wherein R1 and R3 are each independently H, alkyl, preferably C1-6-alkyl, for example C1, C2, C3, C4, C5 or C6-alkyl, more preferably is H or methyl, or - CH2(CH2)ₓ-SH wherein x is 0, or 1 to 6.

14. A molecule comprising or having the chemical structure below: 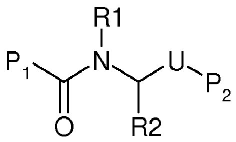
wherein -U- is an aliphatic group optionally substituted, or an aromatic group optionally substituted, or -C(O)-, -O-, -S-, or a phosphorus containing function;
wherein R1 is -CH2(CH2)x-SH wherein x is 0, or 1 to 6, preferably -CH2-CH2-SH; or H or an aliphatic group, preferably selected from -CH₃, -CH₂CH₃, -CH(CH₃), and -C(CH₃)₃;
wherein R2 is -H, -CH2(CH2)x-SH wherein x is 0, or 1 to 6, preferably -CH2-SH, or is H or an amino acid side chain;
wherein at least one of R1 and R2 is -CH2(CH2)ₓ-SH wherein x is 0, or 1 to 6, and
wherein P1 and P2 are as defined in any one of claims 1 to 9.

15. A molecule of claim 14 comprising or having the chemical structure below: 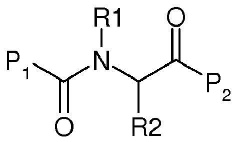 wherein R1, R2, P1 and P2 are as defined in claim 14.
